# EUROPEAN PATENT SPECIFICATION

(11) **EP 1 257 292 B1**
(45) Date of publication and mention of the grant of the patent: **27.04.2011**
(21) Application number: 01911662.3
(22) Date of filing: 20.02.2001
(51) Int. Cl.: A61K 38/17

(54) **USE OF IL-18 INHIBITORS**
VERWENDUNG VON IL-18 INHIBITOREN
UTILISATION D'INHIBITEURS DE IL-18

(30) Priority: 21.02.2000 EP 00103590; 21.02.2000 EP 00103597; 04.10.2000 EP 00121651; 23.11.2000 EP 00125633
(43) Date of publication of application: 20.11.2002
(73) Proprietor: Merck Serono SA, 1267 Coinsins, Vaud (CH); YEDA RESEARCH AND DEVELOPMENT CO., LTD., 76100 Rehovot (IL)
(72) Inventor: CHVATCHKO, Yolande, CH-1232 Confignon (CH); DINARELLO, Charles, Boulder, CO 80302 (US); PLATER-ZYBERK, Christine, CH-1227 Geneva (CH); VAN DEVENTER, Santer, NL-2012 CH Haarlem (NL); RUBINSTEIN, Menachem, 54042 Givat Shmuel (IL); NOVICK, Daniela, 76302 Rehovot (IL); KIM, Soo-Hyun, Denver, CO 80224 (US)
(74) Representative: Merck Serono SA - Geneva Intellectual Property
(86) International application number: PCT/EP2001/001867
(87) International publication number: WO 2001/062285

(56) References cited:
- EP-A- 0 864 585
- EP-A- 0 974 600
- WO-A-98/22137
- WO-A-99/09063
- H. TSUTSUI ET AL.: "IL-18 accounts for both TNF-alpha- and Fas ligand-mediated hepatotoxic pathways in endotoxin-induced liver injury in mice." THE JOURNAL OF IMMUNOLOGY, vol. 159, no. 8, 15 October 1997 (1997-10-15), pages 3961-3967, XP002141329 Baltimore, MD, USA
- S. MCCARTNEY ET AL.: "Selective COX-2 inhibitors and human inflammatory bowel disease." ALIMENTARY PHARMACOLOGY AND THERAPEUTICS, vol. 13, no. 8, August 1999 (1999-08), pages 1115-1117, XP002168245 Oxford, GB
- C. DINARELLO: "Targeting interleukin 18 with interleukin 18 binding protein." ANNALS OF THE RHEUMATIC DISEASES, vol. 59, no. suppl. 1, November 2000 (2000-11), pages I17-I20, XP000973457 London, GB

## Description

### FIELD OF THE INVENTION

The present disclosure relates to the therapeutical use of IL-18 inhibitors in the treatment and/or prevention of alcoholic hepatitis.

### BACKGROUND OF THE INVENTION

In 1989, an endotoxin-induced serum activity that induced interferon-γ (IFN-γ) obtained from mouse spleen cells was described (Micallef et al., 1996). This serum activity functioned not as a direct inducer of IFN-γ but rather as a co-stimulant together with IL-2 or mitogens. An attempt to purify the activity from post-endotoxin mouse serum revealed an apparently homogeneous 50-55 kDa protein. Since other cytokines can act as co-stimulants for IFN-γ production, the failure of neutralizing antibodies to IL-1, IL-4, IL-5, IL-6, or TNF to neutralize the serum activity suggested it was a distinct factor. In 1995, the same scientists demonstrated that the endotoxin-induced co-stimulant for IFN-γ production was present in extracts of livers from mice preconditioned with P.acnes (Novick et al., 1992). In this model, the hepatic macrophage population (Kupffer cells) expand and in these mice, a low dose of bacterial lipopolysaccharide (LPS), which in non-preconditioned mice is not lethal, becomes lethal. The factor, named IFN-γ-inducing factor (IGIF) and later designated interleukin-18 (IL-18), was purified to homogeneity from 1,200 grams of P. acnes-treated mouse livers. Degenerate oligonucleotides derived from amino acid sequences of purified IL-18 were used to clone a murine IL-18 cDNA (Novick et al., 1992). IL-18 is an 18-19 kDa protein of 157 amino acids, which has no obvious similarities to any peptide in the databases. Messenger RNAs for IL-18 and interleukin-12 (IL-12) are readily detected in Kupffer cells and activated macrophage. Recombinant IL-18 induces IFN-gamma more potently than does IL-12, apparently through a separate pathway (Novick et al., 1992). Similar to the endotoxin-induced serum activity, IL-18 does not induce IFN-γ by itself, but functions primarily as a co-stimulant with mitogens or IL-2. IL-18 enhances T cell proliferation, apparently through an IL-2-dependent pathway, and enhances Th1 cytokine production in vitro and exhibits synergism when combined with IL-12 in terms of enhanced IFN-γ production (Maliszewski et al., 1990).

Neutralizing antibodies to mouse IL-18 were shown to prevent the lethality of low-dose LPS in P. acnes pre-conditioned mice. Others had reported the importance of IFN-γ as a mediator of LPS lethality in pre-conditioned mice. For example, neutralizing anti-IFN-γ antibodies protected mice against Shwartzman-like shock (Fantuzzi et al., 1998), and galactosamine-treated mice deficient in the IFN-γ receptor were resistant to LPS-induced death (Bym, 1990). Hence, it was not unexpected that neutralizing antibodies to murine IL-18 protected P. acnes-preconditioned mice against lethal LPS (Novick et al., 1992). Anti-murine IL-18 treatment also protected surviving mice against severe hepatic cytotoxicity.

After the murine form was cloned, the human cDNA sequence for IL-18 was reported in 1996 (Okamura et al., 1995). Recombinant human IL-18 exhibits natural IL-18 activity (Okamura et al., 1995). Human recombinant IL-18 is without direct IFN-γ-inducing activity on human T-cells, but acts as a co-stimulant for production of IFN-γ and other T-helper cell-1 (Th1) cytokines (Okamura et al., 1995). To date, IL-18 is thought of primarily as a co-stimulant for Th1 cytokine production (IFN-γ, IL-2 and granulocyte-macrophage colony stimulating factor) (Izaki, 1978) and also as a co-stimulant for FAS ligand-mediated cytotoxicity of murine natural killer cell clones (Novick et al., 1989).

By cloning IL-18 from affected tissues and studying IL-18 gene expression, a close association of this cytokine with an autoimmune disease was found. The non-obese diabetic (NOD) mouse spontaneously develops autoimmune insulitis and diabetes, which can be accelerated and synchronized by a single injection of cyclophosphamide. IL-18 mRNA was demonstrated by reverse transcriptase PCR in NOD mouse pancreas during early stages of insulitis. Levels of IL-18 mRNA increased rapidly after cyclophosphamide treatment and preceded a rise in IFN-γ mRNA, and subsequently diabetes. Interestingly, these kinetics mimic that of IL-12-p40 mRNA, resulting in a close correlation of individual mRNA levels. Cloning of the IL-18 cDNA from pancreas RNA followed by sequencing revealed identity with the IL-18 sequence cloned from Kupffer cells and in vivo pre-activated macrophages. Also NOD mouse macrophages responded to cyclophosphamide with IL-18 gene expression while macrophages from Balb/c mice treated in parallel did not. Therefore, IL-18 expression is abnormally regulated in autoimmune NOD mice and closely associated with diabetes development (Novick et al., 1992).

IL-18 plays a potential role in immunoregulation or in inflammation by augmenting the functional activity of Fas ligand on Th1 cells (Conti et al., 1997). IL-18 is also expressed in the adrenal cortex and therefore might be a secreted neuro-immunomodulator, playing an important role in orchestrating the immune system following a stressful experience (Chater, 1986).

In vivo, IL-18 is formed by cleavage of pro-IL-18, and its endogenous activity appears to account for IFN-γ production in P. acnes and LPS-mediated lethality. Mature IL-18 is produced from its precursor by the IL-10 converting enzyme (IL-1beta-converting enzyme, ICE, caspase-1).

The IL-18 receptor consists of at least two components, co-operating in ligand binding. High- and low-affinity binding sites for IL-18 were found in murine IL-12 stimulated T cells (Yoshimoto et al., 1998), suggesting a multiple chain receptor complex. Two receptor subunits have been identified so far, both belonging to the IL-1 receptor family (Pamet et al., 1996). The signal transduction of IL-18 involves activation of NF-κB (DiDonato et al., 1997).

Several known cytokine binding proteins are soluble cytokine receptors and correspond to the extracellular ligand binding domains of their respective cell surface cytokine receptors. They are derived either by alternative splicing of a pre-mRNA, common to the cell surface receptor, or by proteolytic cleavage of the cell surface receptor. Such soluble receptors have been described in the past, including among others, the soluble receptors of IL-6 and IFN-γ (Nakamuraet al., 1989), TNF (Dao et al., 1996; Engelmann et al., 1989), IL-1 and IL-4 (John, 1986), IFN-α/β (Mizushima and Nagata, 1990) and others. One cytokine-binding protein, named osteoprotegerin (OPG, also known as osteoclast inhibitory factor - OCIF), a member of the TNFR/Fas family, appears to be the first example of a soluble receptor that exists only as a secreted protein (Anderson, 1997; Bollon, 1980).

Recently, soluble protein having a high affinity for IL-18 has been isolated from human urine, and the human and mouse cDNAs were described (Novick et al., 1999; WO 99/09063). The protein has been designated IL-18 binding protein (IL-18BP).

IL-18BP is not the extracellular domain of one of the known IL18 receptors, but a secreted, naturally circulating protein. It belongs to a novel family of secreted proteins. The family further includes several Poxvirus-encoded proteins which have a high homology to IL-18BP (Novick et al., 1999). IL-18BP is constitutively expressed in the spleen, belongs to the immunoglobulin superfamily, and has limited homology to the IL-1 type II receptor. Its gene was localized on human chromosome 11q13, and no exon coding for a transmembrane domain was found in an 8.3 kb genomic sequence (Novick et al., 1999).

Four human and two mouse isoforms of IL-18BP, resulting from mRNA splicing and found in various cDNA libraries and have been expressed, purified, and assessed for binding and neutralization of IL-18 biological activities (Kim et al., 2000). Human IL-18BP isoform a (IL-18BPa) exhibited the greatest affinity for IL-18 with a rapid on-rate, a slow off-rate, and a dissociation constant (K(d)) of 399 pM. IL-18BPc shares the Ig domain of IL-18BPa except for the 29 C-terminal amino acids; the K(d) of IL-18BPc is 10-fold less (2.94 nM). Nevertheless, IL-18BPa and IL-18BPc neutralize IL-18 >95% at a molar excess of two. IL-18BPb and IL-18BPd isoforms lack a complete Ig domain and lack the ability to bind or neutralize IL-18. Murine IL-18BPc and IL-18BPd isoforms, possessing the identical Ig domain, also neutralize >95% murine IL-18 at a molar excess of two. However, murine IL-18BPd, which shares a common C-terminal motif with human IL-18BPa, also neutralizes human IL-18. Molecular modelling identified a large mixed electrostatic and hydrophobic binding site in the Ig domain of IL-18BP, which could account for its high affinity binding to the ligand (Kim et al., 2000).

Recently, it has been suggested that the interleukin IL-18 is involved in the progression of pathogenicity in chronic inflammatory diseases, including endotoxin shock, hepatitis, and autoimmune-diabetes (Kahiwamura and Okamura, 1998). A further indication of a possible role of IL-18 in the development of liver injury resulted from experiments published by Tsuij et al. (Tsuij et al., 1999), showing an elevated level of IL-18 in lipopolysaccharide-induced acute liver injury in a mouse model. However, the mechanism of the multi-functional factor IL-18 in the development of liver injury has not been elucidated so far.

Liver damage or injury may have diverse causes. It may be due to viral or bacterial infections, alcohol abuse, immunological disorders, or cancer, for example.

Viral hepatitis, due to Hepatitis B virus and Hepatitis C virus, for example, are poorly managed diseases that afflict large number of people world-wide. The number of known hepatitis viruses known is constantly increasing. Apart from Hepatitis B and C virus, at least four other viruses causing virus-associated hepatitis have been discovered so far, called Hepatitis A, D, E and G-Virus.

Alcoholic liver disease is another widespread disease associated with chronic consumption of alcohol. Immune hepatitis is a rare autoimmune disease that is poorly managed. Liver injury also includes damages of the bile ducts. Primary biliary cirrhosis (PBC) is an autoimmune liver disease characterized by destruction of the intrahepatic bile ducts.

Several studies have demonstrated that damage to the liver in diseases such as alcoholic hepatitis, liver cirrhosis, viral hepatitis and primary biliary cirrhosis is associated with T-helper cell-1 (Th1) responses. In one study, a novel liver injury model was established in mice by targeting of ovalbumin-containing liposomes into the liver, followed by adoptive transfer of ovalbumin-specific Th1 cells. Combined treatment of mice with ovalbumin-containing liposomes and Th1 cell transfer caused an increase in serum transaminase activity that was paralleled with an elevation of serum IFN-γ levels. In sharp contrast, ovalbumin-specific Th2 cell transfer resulted in an increase of serum IL-4 levels but did not induce liver injury. The liver injury was blocked by anti-IFN-γ antibodies and anti-tumor necrosis factor (TNF)-α antibodies. These findings indicate that Th1 cells are the major effector cells in acute liver injury (Nishimura and Ohta, 1999) In another set of studies it was shown that mice over-expressing IFN-γ exhibit spontaneous hepatitis without any pathogen or any other stimulant (Okamoto et al., 1998).

Another study implicated Th1 responses in primary biliary cirrhosis (PBC). PBC is an autoimmune liver disease characterized by destruction of the intrahepatic bile ducts. It is generally believed that cellular immune mechanisms, particularly involving T cells, result in this bile duct damage. The relative strength of Th1 and Th2 responses has recently been proposed to be an important factor in the pathophysiology of various autoimmune diseases. In this study, the subset balance in PBC was evaluated by detection of cytokines specific to the two T-cell subsets, i.e., IFN-γ for Th1 cells and IL-4 for Th2 cells. IFN-γ and IL-4 messenger RNA (mRNA) positive cells were counted in liver sections from 18 patients with PBC and 35 disease controls including chronic active hepatitis C, extrahepatic biliary obstruction, and normal liver, using nonisotopic in situ hybridization and immunohistochemistry. Mononuclear cells expressing IFN-γ and IL-4 mRNA were aggregated in inflamed portal tracts in PBC livers, but were rarely present in extrahepatic biliary obstruction, alcoholic fibrosis, or normal liver sections. The IFN-γ and IL-4 mRNA positive cells in PBC livers were detected in significantly higher numbers than in control livers (P < 0,01). Moreover, IFN-γ mRNA expression was more commonly detected than IL-4 expression in PBC livers, and the levels of IFN-γ mRNA expression were highly correlated with the degree of portal inflammatory activity. IFN-γ mRNA-positive cells were detected primarily around damaged bile ducts that were surrounded by lymphoid aggregates. The data indicate that Th1 cells are the more prominent T-cell subset in the lymphoid infiltrates in PBC (Harada et al., 1997).

The cytokine pattern on viral antigen recognition is also believed to exert a profound influence on the resolution of viral infections and viral clearance. One study investigated whether a cytokine imbalance oriented toward Th2 type response plays a role in chronic hepatitis B. Cytokine profiles of peripheral blood mononuclear cells associated with chronic hepatitis B were analyzed by RT-PCR. Upon hepatitis B surface antigen (HbsAg) stimulation, expression of IFN-γ, IL-2, IL-4, and IL-10 was detected in 41%, 8%, 41%, and 50% of the patients, respectively. Among these cytokines, the expression of the Th1 cytokine IFN-γ was associated with high levels of serum AST/ALT (Aspartate aminotransferase/Alanine aminotransferase), representing typical markers of liver damage. Th2 type cytokines were not shown to exert a protective effect on hepatocytes. In conclusion, production of a Th1 cytokine, IFN-γ, by HBsAg-reactive cells was associated with hepatocyte damage in chronic hepatitis B (Lee et al., 1999). High levels of the FAS ligand and its receptor (CD95) were reported in liver of hepatitis B patients (Luo et al., 1997). FAS ligand is considered to be one of the major cytotoxic agents leading to hepatocyte apoptosis.

Another study identified factors associated with the progression of liver injury in 30 hepatitis C virus/RNA (HCV/RNA)-positive untreated patients with chronic hepatitis. Necroinflammatory and architectural damage were evaluated using Ishak's score. Activated hepatic stellate cells (HSC) were visualized by immunohistochemistry for α-smooth muscle actin (αSMA) and quantitated by morphometry. Plasma HCV/RNA was evaluated using a competitive RT-PCR method. To study the type of immune response involved in the progression of liver injury, IFN-γ-positive cells (as expression of a Th1-like response) were evaluated by immunohistochemistry and quantitated by morphometry. It was found that HSC were mostly detected close to areas of lobular necroinflammation or lining fibrotic septa. The αSMA- and Sirius Red-positive parenchyma correlated significantly with necroinflammatory and architectural scores. IFNγ-positive cells were detected in periportal areas associated with the inflammatory infiltrates and significantly correlated with architectural damage. It was therefore concluded that HSC activation and progression of liver injury are associated with a Th1-like response (Baroni et al, 1999). Similarly to the case of Hepatitis B, FAS ligand and its receptor were found in liver and sera of hepatitis C patients (Hiramatsu et al, 1994; Okazaki et al, 1996; Lio et al., 1998)

Th1 cytokines and other Th1 markers were found to be associated with alcoholic hepatitis and liver cirrhosis. Inflammatory stimuli and lipid peroxidation activate nuclear factor κB (NF-κB) and upregulate proinflammatory cytokines and chemokines. In one study, the relationship between pathological liver injury, endotoxemia, lipid peroxidation, and NF-κB activation and imbalance between pro- and anti-inflammatory cytokines was evaluated. Rats (5 per group) were fed ethanol and a diet containing saturated fat, palm oil, corn oil, or fish oil by intragastric infusion. Dextrose isocalorically replaced ethanol in control rats. Pathological analysis was performed and measurements of endotoxin were taken, lipid peroxidation, NF-κB. and messenger RNA (mRNA) levels of proinflammatory cytokines (TNFα, IL-1beta, IFN-γ, and IL-12), C-C chemokines (regulated upon activation, normal T cell expressed and secreted [RANTES], monocyte chemotactic protein [MCP]-1, macrophage inflammatory protein [MIP]-1-α), C-X-C chemokines (cytokine induced neutrophil chemoattractant [CINC], MIP-2, IP-10, and epithelial neutrophil activating protein [ENA]-78), and anti-inflammatory cytokines (IL-10, IL-4, and IL-13). Activation of NF-κB and increased expression of proinflammatory cytokines C-C and C-X-C chemokines was seen in the rats exhibiting necroinflammatory injury (fish oil-ethanol and corn oil-ethanol). These groups also had the highest levels of endotoxin and lipid peroxidation. Levels of IL-10 and IL-4 mRNA were lower in the group exhibiting inflammatory liver injury. Thus, activation of NF-κB occurs in the presence of proinflammatory stimuli and results in increased expression of Th1 proinflammatory cytokines and chemokines (Naji et al., 1999). FAS ligand and its receptor are also elevated in alcoholic liver diseases, suggesting once again that Th1 cytokines are involved in the autoimmune processes induced in alcoholic hepatitis (Galle et al., 1995; Taieb et al, 1998; Fiore et al., 1999).

TNF-α has also emerged as a common pathway in the pathogenesis of alcohol-related hepatic necro-inflammation. Increased levels of hepatic and serum TNF have been documented in animal models of alcoholic liver disease and in human alcoholic liver disease. This dysregulated TNF metabolism has been postulated to play a role in many of the metabolic complications and the liver injury of alcoholic liver disease (Grove et al., 1997; McClain and Cohen, 1989). For instance it was found in one study that patients with alcoholic hepatitis had higher TNF-α levels (mean, 26.3 ng/L; 95% Cl, 21.7 to 30.9) than normal subjects (6.4 ng/L; Cl, 5.4 to 7.4). Patients who subsequently died had a higher TNF-α level (34.7 ng/L; Cl, 27.8 to 41.6) than survivors (16.6 ng/L; Cl, 14.0 to 19.2). In patients with alcoholic hepatitis, TNF-α levels correlated positively with serum bilirubin (r = 0.74; P = 0.0009) and serum creatinine (r = 0.81; P = 0.0003). Patients with alcoholic hepatitis had higher TNF-α levels than patients with inactive alcoholic cirrhosis (11.1 ng/L; Cl, 8.9 to 13.3) and severely alcoholic persons without liver disease (6.4 ng/L; Cl, 5.0 to 7.8). Patients with abnormal renal function had lower TNF-α levels (14.1 ng/L; Cl, 5.4 to 22.8) than patients with alcoholic hepatitis. It was therefore concluded that elevations in TNF-α in alcoholic hepatitis are most marked in severe cases, suggesting that TNF-α plays a role in the pathogenesis (Bird et al., 1990)

TNF mediates many of the biologic actions of endotoxin. Recent studies have shown that TNF administration may cause liver injury and that TNF may mediate the lethality of the hepatotoxin galactosamine. One of the most potent TNF inducers is endotoxin. Because patients with alcoholic liver disease frequently have endotoxemia and because many of the clinical manifestations of alcoholic hepatitis are known biologic actions of TNF, its activity was evaluated in patients with alcoholic hepatitis. Basal and lipopolysaccharide-stimulated TNF release from peripheral blood monocytes, a major source of TNF production, was determined in 16 patients with alcoholic hepatitis and 16 healthy volunteers. Eight of 16 alcoholic hepatitis patients and only two of 16 healthy volunteers had detectable spontaneous TNF activity (p less than 0.05). After lipopolysaccharide stimulation, mean monocyte TNF release from alcoholic hepatitis patients was significantly increased to over twice that of healthy controls (25.3 +/- 3.7 vs. 10.9 +/- 2.4 units per ml, p less than 0.005). It was therefore concluded that monocytes from alcoholic hepatitis patients have significantly increased spontaneous and lipopolysaccharide-stimulated TNF release compared to monocytes from healthy volunteers (McClain and Cohen, 1989.

Lipopolysaccharide (LPS)-binding protein (LBP) and CD14 play key intermediary roles in the activation of cells by endotoxin. Gut-derived LPS has been postulated to participate in promoting pathological liver injury in alcoholic liver disease. It was demonstrated that rats fed intragastrically with ethanol in oil for 4 weeks had elevated levels of CD14 and LBP in their Kupffer cells and hepatocytes, respectively. Expression of CD14 mRNA was also elevated in nonmyeloid cells. Enhanced LBP and CD14 expression rapidly increases the LPS-induced expression of various pro-inflammatory cytokines and correlates with the presence of pathological liver injury in alcoholic liver injury (Su et al., 1998; Lukkari et al., 1999).

### SUMMARY OF THE INVENTION

The present disclosure is based on the finding that inhibitors of IL-18 are effective for treatment and/or prevention of alcoholic hepatitis.

The disclosure therefore relates to the use of an IL-18 inhibitor for the manufacture of a medicament for treatment and/or prevention of alcoholic hepatitis.

The use of combinations of an IL-18 inhibitor are also considered according to the diclosure. In order to apply gene therapeutical approaches to deliver the IL-18 inhibitor to diseased tissues or cells, further aspects of the disclosure relate to the use of expression vectors comprising the coding sequence of an IL-18 inhibitor for the treatment and/or prevention of the disease conditions. The disclosure further relates to the use of endogenous gene activation of IL-18 inhibitors and to the use of cells genetically engineered to express IL-18 inhibitors for the prevention and/or treatment of alcoholic hepatitis.

### BRIEF DESCRIPTION OF THE DRAWINGS

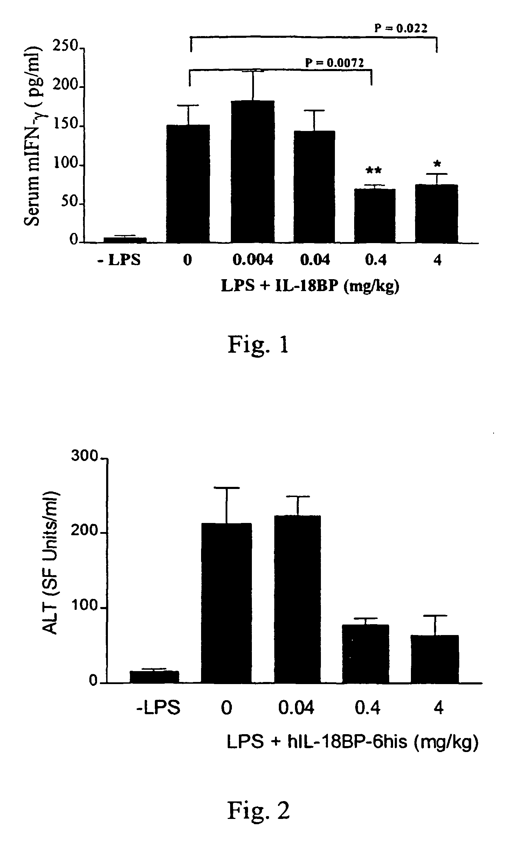

- Fig.1: shows a histogram depicting the serum levels of IFN-γ (pg/ml) after injection of various amounts of recombinant IL18BP (0; 0,04; 0,4; 4 mg/kg) into mice 1h before the injection of LPS. Blood samples were taken 5 h after LPS injection and analyzed by ELISA for circulating IFN-γ.
- Fig. 2: shows a histogram depicting the serum levels of Alanine aminotransferase (ALT). Mice were injected with increasing doses of recombinant human IL18BP (0; 0,04; 0,4; 4 mg/kg) before injection of LPS into P. acnes sensitized mice. Blood samples were taken 5h after LPS injection and serum levels of ALT were measured. SF = Sigma-Frankel: 1 SF Unit of AST/ALT will form 4,82x10⁻⁴ µmol glutamate/minute at pH 7.5 at 25°C.

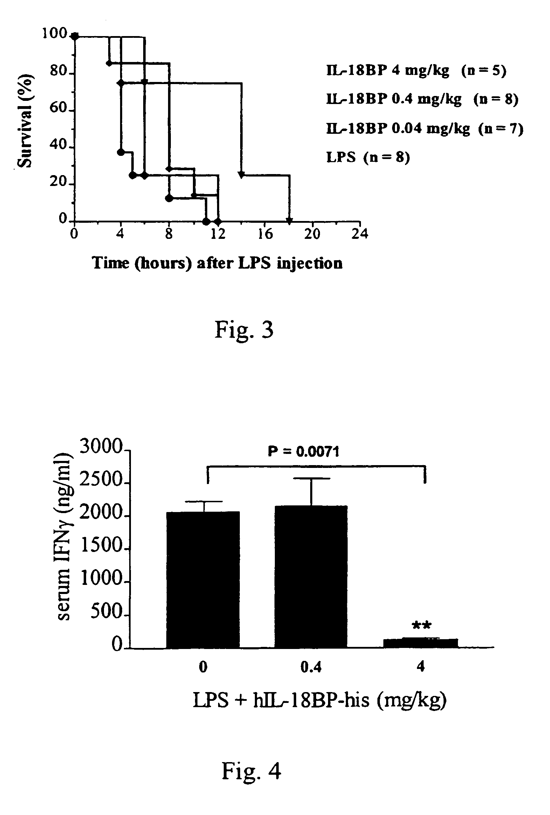

- Fig. 3: shows the survival time of the mice after LPS injection. Mice were injected with different doses of recombinant human IL18BP (0; 0,04; 0,004; 4 mg/kg) 20 min before injection of LPS into P. acnes sensitized mice. Triangles: 4 mg/kg; small diamond: 0,4; big diamond: 0,04; circles: no IL18BP (only LPS).
- Fig. 4: shows a histogram depicting serum levels of IFN-γ, measured 5 h after injection of different amounts of IL18BP (0; 0,4; 4 mg/kg), which was administered 20 min before LPS injection into P. acnes sensitized mice.

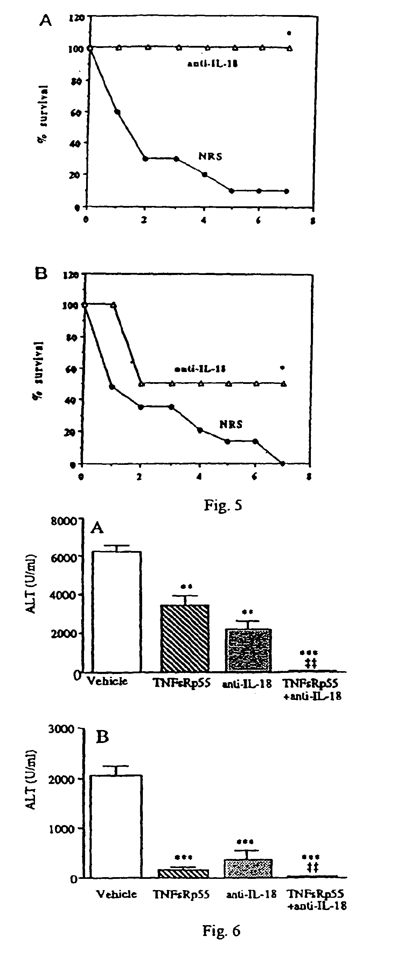

- Fig. 5: shows the survival of mice injected either with polyclonal IL-18 antiserum or normal rabbit serum (NDS = control) 30 min before injection with 40 mg/ml (lethal dosis) of LPS derived from E. coli (Fig. 5 A) or S. thyphimurium (Fig. 5 B). Triangles: mice were injected with IL-18 antiserum; circles: mice were injected with NDS. On the x-axis, the days after LPS challenge are depicted. * p<0,05.
- Fig. 6: shows a histogram, depicting the mean + SEM of five mice per group treated in the following way. Mice were injected intraperitoneally (i.p.) either anti-IL-18 antiserum, soluble TNF-α receptors (TNFsRp55) or vehicle (saline), immediately followed by the intravenous (i.v.) administration of Concanavalin A (Con A; Fig. 6 A) or PEA (Pseudomonas aeruginosa, Fig. 6 B). **p<0,01; ***p<0,001 vs ConA or PEA alone; # p<0,01 vs either TNFsRp55 or anti-IL-18 factorial ANOVA.

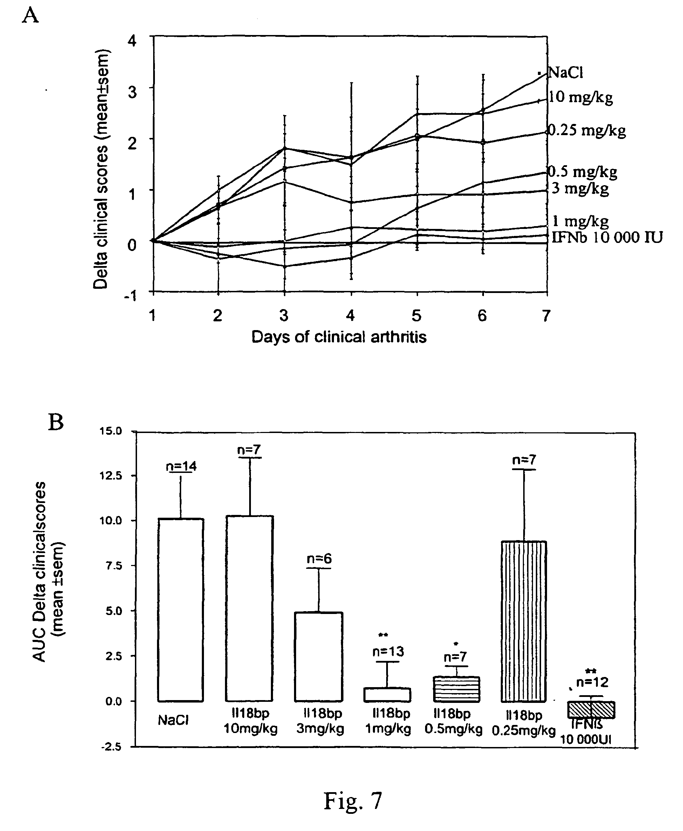

- Fig. 7: shows the effect of of IL-18BP on clinical scores in a murine model of arthritis. Fig. 7 A shows a diagram depicting the clinical scores measured after daily administration of different amounts of IL-18BP or IFN-β or vehicle (NaCl) i.p. (intraperitoneally) to mice. Symbols: Filled triangles: 10 000 IU IFN-β; open triangles: 10 mg/kg IL-18BP, reversed triangles: 3 mg/kg IL-18BP, diamonds: 1 mg/kg IL-18BP; circles: 0.5 mg/kg IL-18BP; open squares: 0.25 mg/kg IL-18BP, and filled squares: NaCl. The days of treatment are depicted on the x-axis, the clinical scores (mean values) are depicted on the y-axis. Statistics were calculated by the Mann Whitney test. Fig. 7 B shows a histogram depicting the AUC (area under the curve) derived from the graph of Fig. 7 A. n = number of animals.

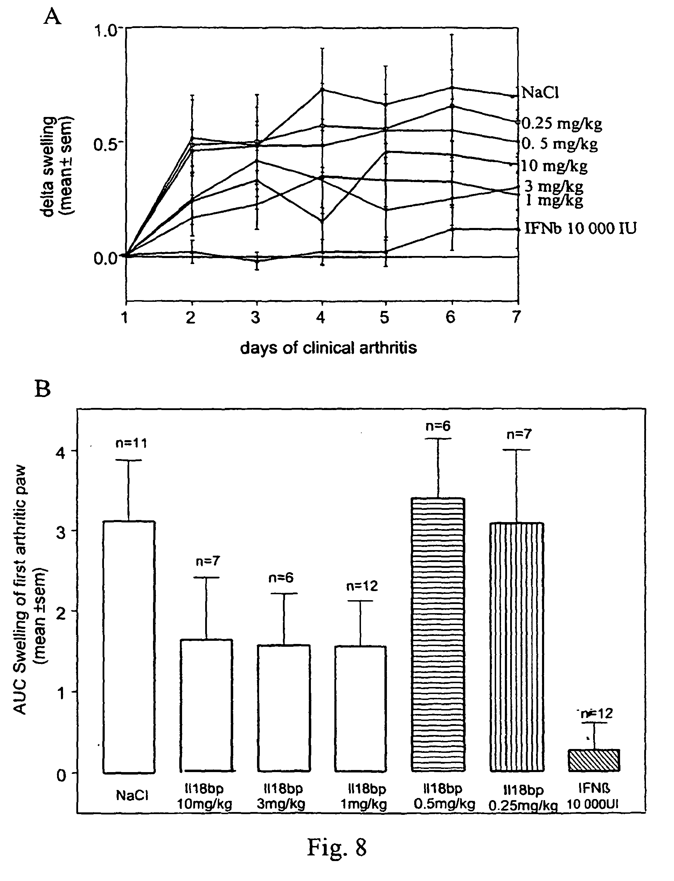

- Fig. 8: shows the effect of IL-18BP on paw swelling. Fig. 8 A shows a diagram depicting the results obtained by measuring the paw thickness (swelling) of diseased hind paws of individual animals treated with different amounts of IL-18BP. The y-axis shows the change of paw thickness in millimeters from the beginning of treatment. The symbols are as in Fig. 7. Fig. 8 B shows a histogram depicting the AUC derived from Fig. 8 A. n = number of animals.

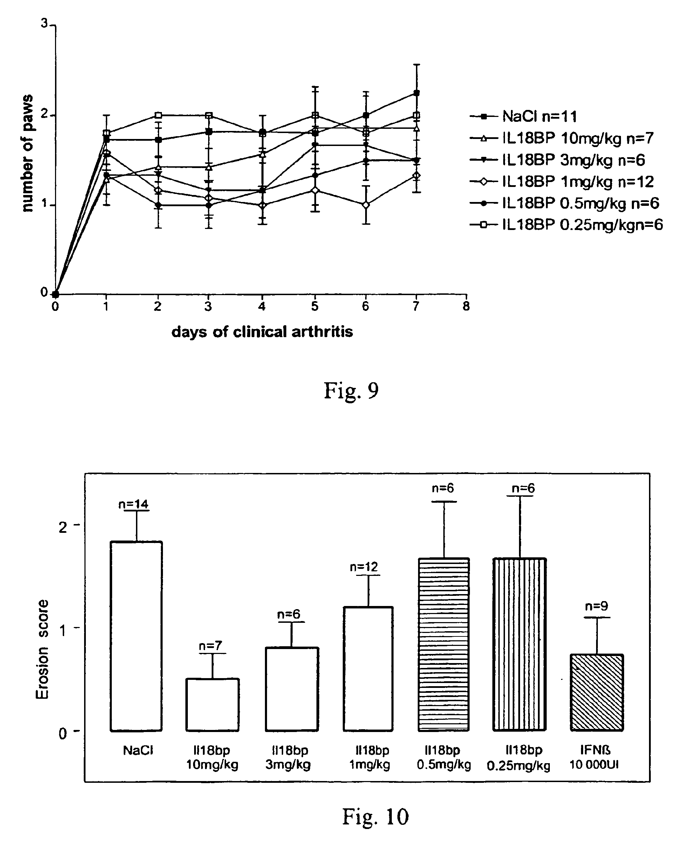

- Fig. 9: shows the analysis of the number of diseased hind paws at the time of acute arthritis, i.e. spreading of the disease to additional joints. Symbols: Filled squares: NaCl (control), triangles: 10 mg/kg IL-18BP, reversed triangles: 3 mg/kg IL-18BP, diamonds: 1 mg/kg IL-18BP, circles: 0.5 mg/kg IL-18BP and open squares: 0.25 mg/kg IL-18BP.
- Fig. 10: shows a histogram depicting the erosion scores of the cartilage of diseased joints.

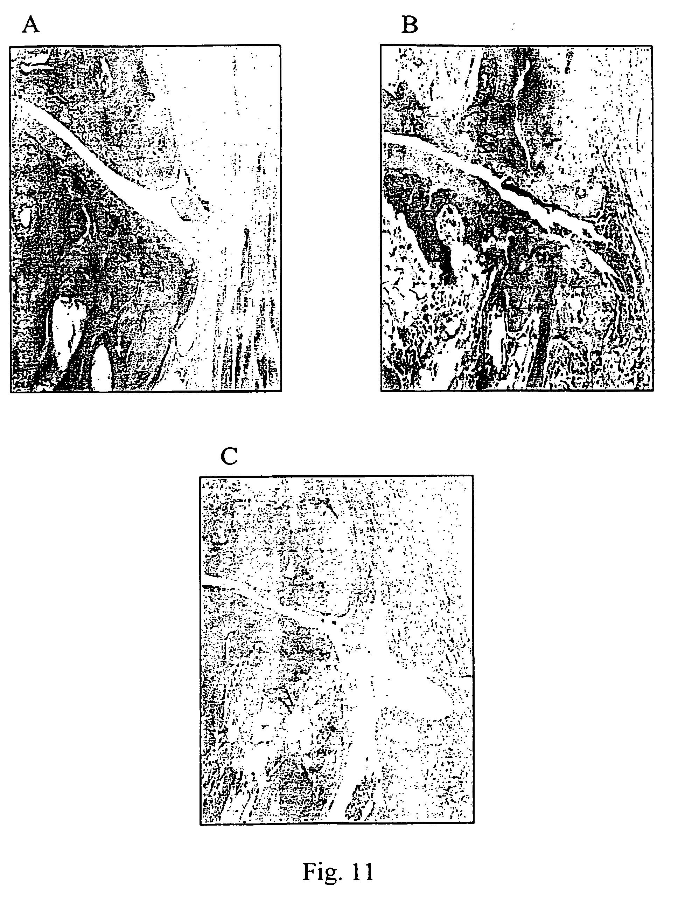

- Fig. 11: shows the histopathology of mouse joints. At the end of the experiment, the paw that first developed arthritis was dissected away, fixed and processed as described in Example 10 below. Fig. 11 A: normal mouse joint; Fig. 11 B: joint from an arthritic mouse; Fig. 11 C: joint from a mouse treated with rhlL-18BP.

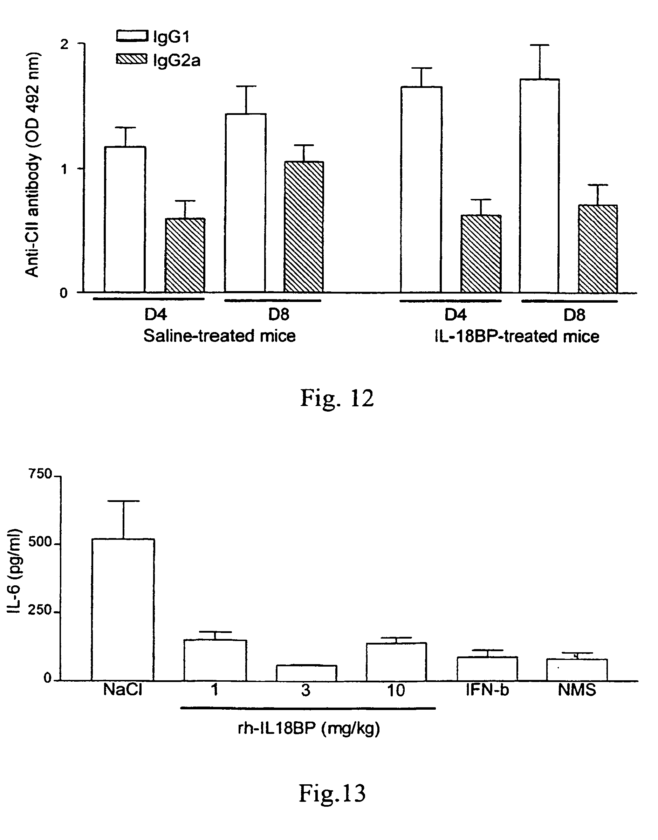

- Fig. 12: shows a histogram depicting the levels of anti-collagen type II antibodies of the isotype IgG1 (open columns) or IgG2a (hatched columns) of mice treated with 3 mg/kg of IL-18BP or saline (vehicle), respectively. Measurements were taken on day 4 (D4) or day 8 (D8) of the disease.
- Fig. 13: shows a histogram depicting IL-6 levels in pg/ml of animals treated with 1, 3 or 10 mg/kg of IL-18BP, 10 000 IU of Interferon β (IFN-b), normal mouse serum (NMS) or saline (NaCl), respectively.

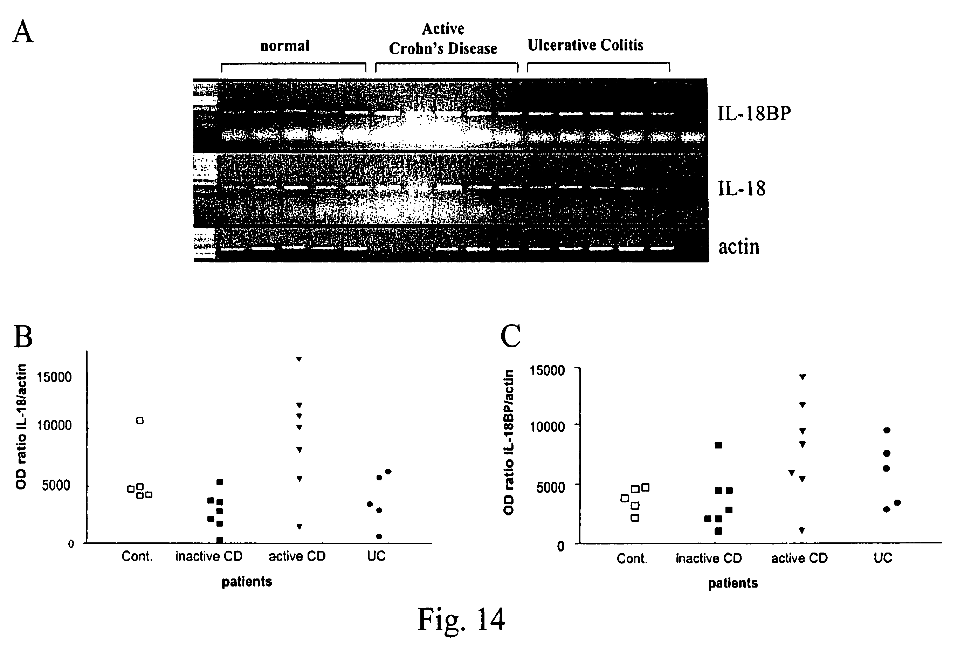

- Fig. 14: shows the expression of hIL-188P and IL-18 mRNA transcripts in intestinal biopsies from patients suffering from active Crohn's disease, ulcerative colitis or normal healthy individuals. Representative RT-PCR products are shown for IL- 18BP, for IL-18 and for a housekeeping gene (β-actin) (Fig. 14 A). Relative quantification of ethidiumbromide-stained bands was carried out using the Kodak Digital Imaging Software and are reported as the ratio of target gene to β-actin. The target gene is IL-18 in Fig. 14 B and IL-18BP in Fig. 14 C.

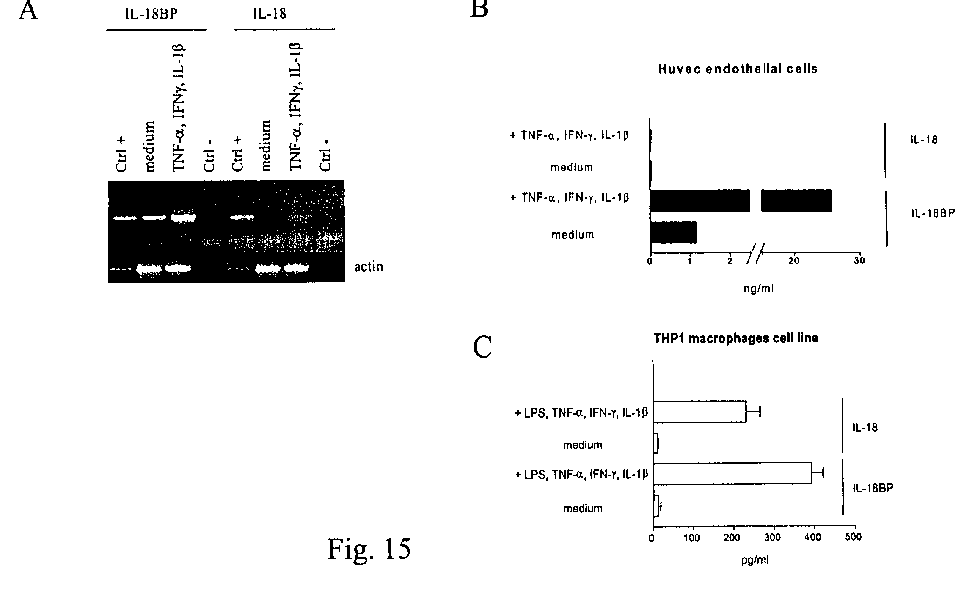

- Fig. 15: shows the expression of hIL-18BP mRNA transcripts and of protein by HUVECs (human umbilical vein endothelial cells) and the expression of protein by THP1 (human monocytic cell line). RNA was isolated from non-treated endothelial cells (medium) and endothelial cells stimulated with IL-1β, TNFα, IFNγ. Positive control: colon from patient with Crohn's disease, negative control: without cDNA. IL-18BP and IL-18 expression was analysed by semiquantitative RT-PCR (Fig. 15 A). Culture supernatant from non-treated (medium) and upon 24h activation with IL-1β, TNFα, IFNγ of HUVEC (Fig. 15 B) or THP1 (Fig. 15 C) cells were analysed for IL-18BP and IL-18 protein production by ELISA.

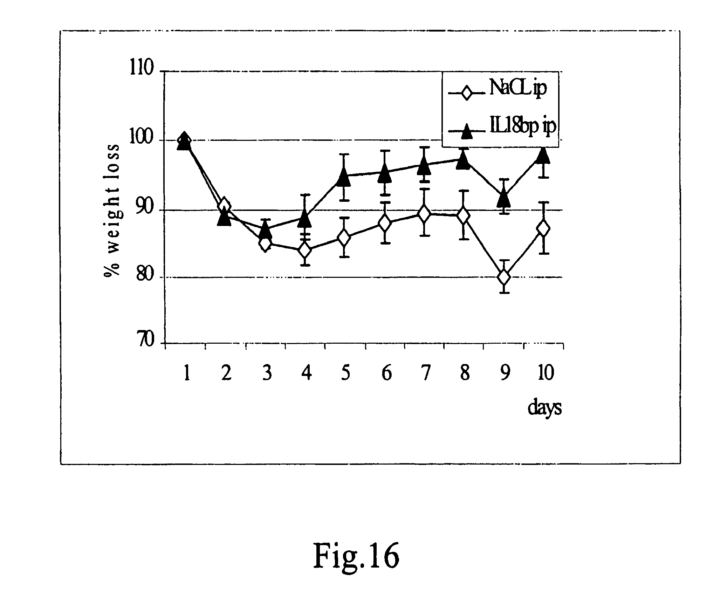

- Fig. 16: shows the development of bodyweight between day 1 and day 10 in a mouse model of IBD after intraperitoneal (ip) administration of either saline (NaCl) or IL- 18BP (8 mg/kg). The change in weight is expressed as percentage of the body weight change from day 1. Mean values and SEM of two groups are shown, 8 mice per group.

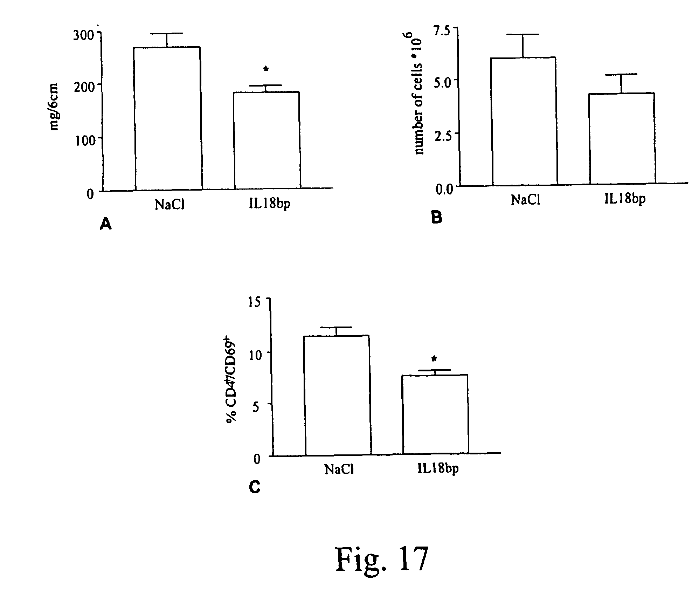

- Fig. 17: shows the results of analyses of colons, caudal lymph nodes and spleen derived from IL-18BP treated vs. non-treated IBD mice. Fig. 17 A depicts the weight of the last 6 centimetres of colon in mg. Fig. 17 B shows the total number of cells present in the caudal lymph node. Fig. 17 C depicts the percentage of cells staining positive for CD4*/CD69* in the spleen. Data represent mean values and SEM. * indicates a significant difference.

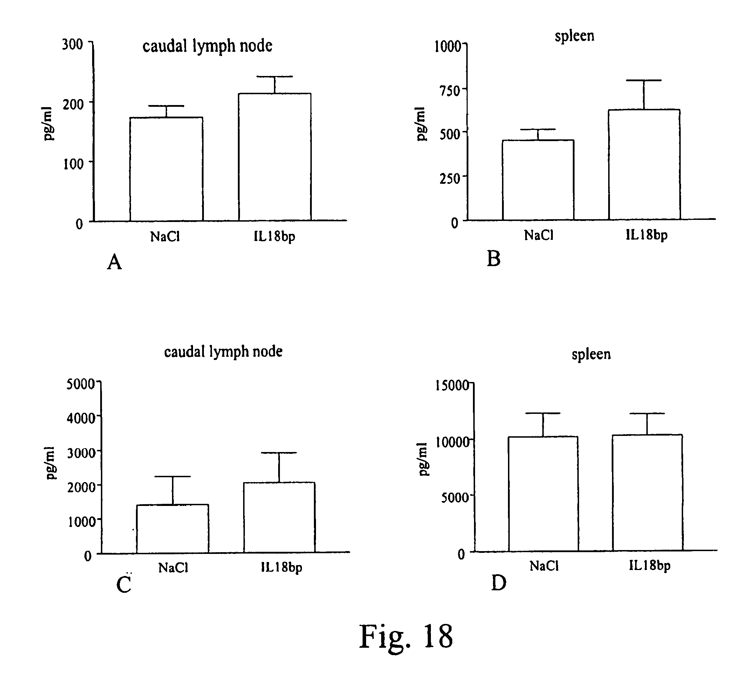

- Fig. 18: shows the amount of IFNγ (Fig. 18 A and B) and TNFα (Fig. 18 C and D) produced after 48 hours by caudal lymph node cells (Fig. 185 A and C) and spleen cells (Fig. 18 B and D) after stimulation with CD3/CD28 present in the supernatants. Mean and SEM are shown.

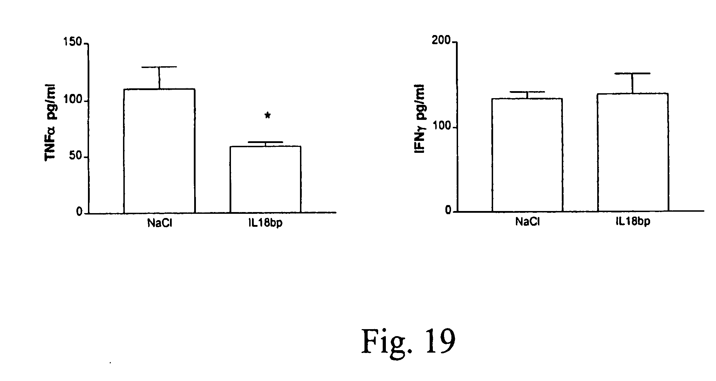

- Fig. 19: shows the TNFα (Fig. 19 A) and IFNγ (Fig. 19 B) content in colon homogenates. Data are corrected for the colon weight. Mean values and SEM are shown. * indicates a significant difference.

### DESCRIPTION OF THE INVENTION

The present disclosure is based on the finding of a beneficial effect of an inhibitor of IL-18 in alcoholic hepatitis.

The term "inhibitor of IL-18" within the context of this disclosure refers to any molecule modulating IL-18 production and/or action in such a way that IL-18 production and/or action is attenuated, reduced, or partially, substantially or completely prevented or blocked. The term "IL-18 inhibitor" is meant to encompass inhibitors of IL-18 production as well as of inhibitors of IL-18 action.

An inhibitor of production can be any molecule negatively affecting the synthesis, processing or maturation of IL-18. The inhibitors considered according to the disclosure can be, for example, suppressors of gene expression of the interleukin IL-18, antisense mRNAs reducing or preventing the transcription of the IL-18 mRNA or leading to degradation of the mRNA, proteins impairing correct folding, or partially or substantially preventing secretion of IL-18, proteases degrading IL-18, once it has been synthesized, inhibitors of proteases cleaving pro-IL-18 in order to generate mature IL-18, such as inhibitors of caspase-1, and the like.

An inhibitor of IL-18 action can be an IL-18 antagonist, for example. Antagonists can either bind to or sequester the IL-18 molecule itself with sufficient affinity and specificity to partially or substantially neutralise the IL-18 or IL-18 binding site(s) responsible for IL-18 binding to its ligands (like, e.g. to its receptors). An antagonist may also inhibit the IL-18 signalling pathway, which is activated within the cells upon IL-18/receptor binding.

Inhibitors of IL-18 action may be also soluble IL-18 receptors or molecules mimicking the receptors, or agents blocking the IL-18 receptors, or IL-18 antibodies, such as polyclonal or monoclonal antibodies, or any other agent or molecule preventing the binding of IL-18 to its targets, thus diminishing or preventing triggering of the intra- or extracellular reactions mediated by IL-18.

According to the present disclosure, inhibitors or IL-18 are used for the manufacture of a medicament for treatment and/or prevention of alcoholic hepatitis.

The term liver injury, or liver disease, a used herein, comprises a variety of different pathological conditions. Several of the conditions contemplated in the present disclosure have been explained in detail in the "Background of the invention" above. Further liver diseases which can be treated and/or prevented according to the disclosure comprise, for example, pyrogenic liver absess. It is also called bacterial liver, and it is a pus-producing cavity within the liver. The causes of a liver abscess are multiple. It can develop from an abdominal infection such as appendicitis, diverticulitis, or a perforated bowel; an infection in the blood; an infection from the biliary (liver secretion) tract; or trauma when a bruised liver becomes infected. The most common organisms causing liver abscess are Escherichia coli, Proteus vulgaris, and Enterobacter aerogenes. The incidence is 1 out of 10,000 people.

Alcoholic liver diseases can be treated and/or prevented using IL-18 inhibitors according to the disclosure. They comprise acute or chronic inflammation of the liver induced by alcohol abuse. Alcoholic hepatitis usually occurs after years of excessive drinking. The longer the duration of alcohol use and the larger the consumption of alcohol, the greater the probability of developing liver disease. Malnutrition develops as a result of empty calories from alcohol, reduced appetite, and malabsorption (inadequate absorption of nutrients from the intestinal tract). Malnutrition contributes to liver disease. The toxicity of ethanol to the liver, individual susceptibility to alcohol-induced liver disease, and genetic factors also contribute to the development of alcoholic liver disease.

In a preferred embodiment of the present disclosure, the inhibitor of IL-18 is selected from inhibitors of caspase-1 (ICE), antibodies directed against IL-18, antibodies directed against any of the IL-18 receptor subunits, inhibitors of the IL-18 signalling pathway, antagonists of IL-18 which compete with IL-18 and block the IL-18 receptor, and IL-18 binding proteins, isoforms, muteins, fused proteins, functional derivatives, active fractions or circularly permutated derivatives thereof having the same activity.

The term "IL-18 binding proteins" is used herein synonymously with "IL18BP". It comprises IL-18 binding proteins as defined in WO 99/09063 or in Novick et al., 1999, including splice variants and/or isoforms of IL-18 binding proteins, as defined in Kim et al., 2000. In particular, human isoforms a and c of IL-18BP are useful in accordance with the present disclosure. The proteins useful according to the present disclosure may be glycosylated or non-glycosylated, they may be derived from natural sources, such as urine, or they may preferably be produced recombinantly. Recombinant expression may be carried out in prokaryotic expression systems like E. coli, or in eukaryotic, and preferably in mammalian, expression systems.

As used herein the term "muteins" refers to analogs of an IL-18BP, or analogs of a viral IL-18BP, in which one or more of the amino acid residues of a natural IL-18BP or viral IL-18BP are replaced by different amino acid residues, or are deleted, or one or more amino acid residues are added to the natural sequence of an IL-18BP, or a viral IL-18BP, without changing considerably the activity of the resulting products as compared with the wild type IL-18BP or viral IL-18BP. These muteins are prepared by known synthesis and/or by site-directed mutagenesis techniques, or any other known technique suitable therefor.

Any such mutein preferably has a sequence of amino acids sufficiently duplicative of that of an IL-18BP, or sufficiently duplicative of a viral IL-18BP, such as to have substantially similar activity to IL-18BP. One activity of IL-18BP is its capability of binding IL-18. As long as the mutein has substantial binding activity to IL-18, it can be used in the purification of IL-18, such as by means of affinity chromatography, and thus can be considered to have substantially similar activity to IL-18BP. Thus, it can be determined whether any given mutein has substantially the same activity as IL-18BP by means of routine experimentation comprising subjecting such a mutein, e.g., to a simple sandwich competition assay to determine whether or not it binds to an appropriately labeled IL-18, such as radioimmunoassay or ELISA assay.

Muteins of IL-18BP polypeptides or muteins of viral IL-18BPs, which can be used in accordance with the present disclosure, or nucleic acid coding therefor, include a finite set of substantially corresponding sequences as substitution peptides or polynucleotides which can be routinely obtained by one of ordinary skill in the art, without undue experimentation, based on the teachings and guidance presented herein.

Preferred changes for muteins in accordance with the present disclosure are what are known as "conservative" substitutions. Conservative amino acid substitutions of IL-18BP polypeptides or proteins or viral IL-18BPs, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g., under thirty, and preferably under ten, and do not remove or displace amino acids which are critical to a functional conformation, e.g., cysteine residues. Proteins and muteins produced by such deletions and/or insertions come within the purview of the present disclosure.

Preferably, the synonymous amino acid groups are those defined in Table I. More preferably, the synonymous amino acid groups are those defined in Table II; and most preferably the synonymous amino acid groups are those defined in Table III.

**TABLE I**

| Preferred Groups of Synonymous Amino Acids | |
|---|---|
| Amino Acid | Synonymous Group |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

**TABLE II**

| More Preferred Groups of Synonymous Amino Acids | |
|---|---|
| Amino Acid | Synonymous Group |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile. Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| Most Preferred Groups of Synonymous Amino Acids | |
|---|---|
| Amino Acid | Synonymous Group |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gin |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of IL-18BP polypeptides or proteins, or muteins of viral IL-18BPs, for use in the present disclosure include any known method steps, such as presented in US patents RE 33,653, 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Shaw et al).

The term "fused protein" refers to a polypeptide comprising an IL-18BP, or a viral IL-18BP, or a mutein or fragment thereof, fused with another protein, which, e.g., has an extended residence time in body fluids. An IL-18BP or a viral IL-18BP, may thus be fused to another protein, polypeptide or the like, e.g., an immunoglobulin or a fragment thereof.

"Functional derivatives" as used herein cover derivatives of IL-18BPs or a viral IL-18BP, and their muteins and fused proteins, which may be prepared from the functional groups which occur as side chains on the residues or the N- or C-terminal groups, by means known in the art, and are included in the disclosure as long as they remain pharmaceutically acceptable, i.e. they do not destroy the activity of the protein which is substantially similar to the activity of IL-18BP, or viral IL-18BPs, and do not confer toxic properties on compositions containing it.

These derivatives may, for example, include polyethylene glycol side-chains, which may mask antigenic sites and extend the residence of an IL-18BP or a viral IL-18BP in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "active fractions" of an IL-18BP, or a viral IL-18BP, muteins and fused proteins, the present disclosure covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has substantially similar activity to IL-18BP.

In a further preferred embodiment of the disclosure, the inhibitor of IL-18 is an IL-18 antibody. Anti-IL-18 antibodies may be polyclonal or monoclonal, chimeric, humanised, or even fully human. Recombinant antibodies and fragments thereof are characterised by high affinity binding to IL-18 *in vivo* and low toxicity. The antibodies which can be used in the disclosure are characterised by their ability to treat patients for a period sufficient to have good to excellent regression or alleviation of the pathogenic condition or any symptom or group of symptoms related to a pathogenic condition, and a low toxicity.

Neutralising antibodies are readily raised in animals such as rabbits, goat or mice by immunisation with IL-18. Immunised mice are particularly useful for providing sources of B cells for the manufacture of hybridomas, which in turn are cultured to produce large quantities of anti-IL-18 monoclonal antibodies.

Chimeric antibodies are immunoglobulin molecules characterised by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (Elliott et al., 1994). Humanised antibodies are immunoglobulin molecules created by genetic engineering techniques in which the murine constant regions are replaced with human counterparts while retaining the murine antigen binding regions. The resulting mouse-human chimeric antibody preferably have reduced immunogenicity and improved pharmacokinetics in humans (Knight et al., 1993).

Thus, in a further preferred embodiment, IL-18 antibody is a humanised IL-18 antibody. Preferred examples of humanized anti-IL-18 antibodies are described in the European Patent Application EP 0 974 600, for example.

In yet a further preferred embodiment, the IL-18 antibody is fully human. The technology for producing human antibodies is described in detail e.g. in WO00176310, WO99/53049, US 6,162,963 or AU5336100. Fully human antibodies are preferably recombinant antibodies, produced in transgenic animals, e.g. xenomice, comprising all or parts of functional human Ig loci.

In a highly preferred embodiment of the present disclosure, the inhibitor of IL-18 is a IL-18BP, or an isoform, a mutein, fused protein, functional derivative, active fraction or circularly permutated derivative thereof. These isoforms, muteins, fused proteins or functional derivatives retain the biological activity of IL-18BP, in particular the binding to IL-18, and preferably have essentially at least an activity similar to IL-18BP. Ideally, such proteins have a biological activity which is even increased in comparison to unmodified IL-18BP. Preferred active fractions have an activity which is better than the activity of IL-18BP, or which have further advantages, like a better stability or a lower toxicity or immunogenicity, or they are easier to produce in large quantities, or easier to purify.

The sequences of IL-18BP and its splice variants/isoforms can be taken from WO99/09063 or from Novick et al., 1999, as well as from Kim et al., 2000.

Functional derivatives of IL-18BP may be conjugated to polymers in order to improve the properties of the protein, such as the stability, half-life, bioavailability, tolerance by the human body, or immunogenicity. To achieve this goal, IL18-BP may be linked e.g. to Polyethlyenglycol (PEG). PEGylation may be carried out by known methods, described in WO 92/13095, for example.

Therefore, in a preferred embodiment of the present disclosure, IL-18BP is PEGylated.

In a further preferred embodiment of the disclosure, the inhibitor of IL-18 is a fused protein comprising all or part of an IL-18 binding protein, which is fused to all or part of an immunoglobulin. The person skilled in the art will understand that the resulting fusion protein retains the biological activity of IL-18BP, in particular the binding to IL-18. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met introduced between the IL-18BP sequence and the immunoglobulin sequence. The resulting fusion protein has improved properties, such as an extended residence time in body fluids (half-life), increased specific activity, increased expression level, or the purification of the fusion protein is facilitated.

In a preferred embodiment, IL-18BP is fused to the constant region of an Ig molecule. Preferably, it is fused to heavy chain regions, like the CH2 and CH3 domains of human IgG1, for example. The generation of specific fusion proteins comprising IL-18BP and a portion of an immunoglobulin are described in example 11 of WP99/09063, for example. Other isoforms of Ig molecules are also suitable for the generation of fusion proteins according to the present disclosure, such as isoforms IgG₂ or IgG₄, or other Ig classes, like IgM or IgA, for example. Fusion proteins may be monomeric or multimeric, hetero- or homomultimeric.

Interferons are predominantly known for inhibitory effects on viral replication and cellular proliferation. Interferon-γ, for example, plays an important role in promoting immune and inflammatory responses. Interferon β (IFN-β, an interferon type I), is said to play an anti-inflammatory role. Studies published by Triantaphyllopoulos et al (1999) indicated that IFN-β has a beneficial effect in the therapy of rheumatoid arthritis, as shown in a mouse model of the disease, the collagen-induced arthritis (CIA) model. This beneficial effect of IFN-β was confirmed in the examples below.

The disclosure also relates to the use of a combination of an inhibitor of IL-18 and an interferon in the manufacture of a medicament for the treatment of arthritis, in particular rheumatoid arthritis.

Interferons may also be conjugated to polymers in order to improve the stability of the proteins. A conjugate between Interferon β and the polyol Polyethlyenglycol (PEG) has been described in WO99/55377, for instance.

In another preferred embodiment of the disclosure, the interferon is Interferons-β (IFN-β), and more preferably IFN-β 1a.

The inhibitor of IL-18 production and/or action is preferably used simultaneously, sequentially, or separately with the interferon.

In yet a further embodiment of the disclosure, an inhibitor of IL-18 is used in combination with a TNF antagonist. TNF antagonists exert their activity in several ways. First, antagonists can bind to or sequester the TNF molecule itself with sufficient affinity and specificity to partially or substantially neutralise the TNF epitope or epitopes responsible for TNF receptor binding (hereinafter termed "sequestering antagonists"). A sequestering antagonist may be, for example, an antibody directed against TNF.

Alternatively, TNF antagonists can inhibit the TNF signalling pathway activated by the cell surface receptor after TNF binding (hereinafter termed "signalling antagonists"). Both groups of antagonists are useful, either alone or together, in combination with an IL-18 inhibitor, in the therapy of arthritis, in particular rheumatoid arthritis.

TNF antagonists are easily identified and evaluated by routine screening of candidates for their effect on the activity of native TNF on susceptible cell lines in vitro, for example human B cells, in which TNF causes proliferation and immunoglobulin secretion. The assay contains TNF formulation at varying dilutions of candidate antagonist, e.g. from 0,1 to 100 times the molar amount of TNF used in the assay, and controls with no TNF or only antagonist (Tucci et al., 1992).

Sequestering antagonists are the preferred TNF antagonists to be used according to the present disclosure. Amongst sequestering antagonists, those polypeptides that bind TNF with high affinity and possess low immunogenicity are preferred. Soluble TNF receptor molecules and neutralising antibodies to TNF are particularly preferred. For example, soluble TNF-RI and TNF-RII are useful in the present disclosure. Truncated forms of these receptors, comprising the extracellular domains of the receptors or functional portions thereof, are more particularly preferred antagonists according to the present disclosure. Truncated soluble TNF type-I and type-II receptors are described in EP914431, for example.

Truncated forms of the TNF receptors are soluble and have been detected in urine and serum as 30 kDa and 40 kDa TNF inhibitory binding proteins, which are called TBPI and TBPII, respectively (Engelmann et al., 1990). The simultaneous, sequential, or separate use of the IL-18 inhibitor with the TNF antagonist and /or an Interferon is preferred, according to the disclosure.

According to the disclosure, TBP I and TBPII are preferred TNF antagonists to be used in combination with an IL-18 inhibitor. Derivatives, fragments, regions and biologically active portions of the receptor molecules functionally resemble the receptor molecules that can also be used in the present disclosure. Such biologically active equivalent or derivative of the receptor molecule refers to the portion of the polypeptide, or of the sequence encoding the receptor molecule, that is of sufficient size and able to bind TNF with such an affinity that the interaction with the membrane-bound TNF receptor is inhibited or blocked.

In a further preferred embodiment, human soluble TNF-RI (TBPI) is the TNF antagonist to be used according to the disclosure. The natural and recombinant soluble TNF receptor molecules and methods of their production have been described in the European Patents EP 308 378, EP 398 327 and EP 433 900.

The IL-18 inhibitor can be used simultaneously, sequentially or separately with the TNF inhibitor. Advantageously, a combination of an IL-18 antibody or antiserum and a soluble receptor of TNF, having TNF inhibiting activity, is used.

In a further preferred embodiment of the disclosure, the medicament further comprises a COX-inhibitor, preferably a COX-2 inhibitor. COX inhibitors are known in the art. Specific COX-2 inhibitors are disclosed in WO 01/00229, for example.

The disclosure further relates to the use of a combination of IL-18 inhibitors and/or interferons and/or TNF antagonists and/or COX-2 inhibitors. The combination is suitable for the for the treatment and/or prevention of arthritis, in particular rheumatoid arthritis, and for the treatment and/or prevention of liver injury and for the treatment and/or prevention of inflammatory bowel disease, in particular Crohn's disease and ulcerative cloitis. The active components may be used simultaneously, sequentially, or separately.

In a preferred embodiment of the present disclosure, the inhibitor of IL-18 is used in an amount of about 0.0001 to 10 mg/kg of body weight, or about 0.01 to 5 mg/kg of body weight or about 0.1 to 3 mg/kg of body weight or about 1 to 2 mg/kg of body weight. In yet a further preferred embodiment, the inhibitor of IL-18 is used in an amount of about 0.1 to 1000 µg/kg of body weight or 1 to 100 µg/kg of body weight or about 10 to 50 µg/kg of body weight.

The disclosure further relates to the use of an expression vector comprising the coding sequence of an inhibitor of IL-18 in the preparation of a medicament for the prevention and/or treatment of arthritic conditions or arthritis, in particular rheumatoid arthritis, for the treatment of liver injury, and for the treatment of inflammatory bowel disease. A gene therapeutical approach is thus used for treating and/or preventing the disease. Advantageously, the expression of the IL-18 inhibitor will then be *in situ*, thus efficiently blocking IL-18 directly in the tissue(s) or cells affected by the disease.

In order to treat and/or prevent arthritis, the gene therapy vector comprising the sequence of an inhibitor of IL-18 production and/or action may be injected directly into the diseased joint, for example, thus avoiding problems involved in systemic administration of gene therapy vectors, like dilution of the vectors, reaching and targetting of of the target cells or tissues, and of side effects.

The use of a vector for inducing and/or enhancing the endogenous production of an inhibitor of IL-18 in a cell normally silent for expression of an IL-18 inhibitor, or which expresses amounts of the inhibitor which are not sufficient, are also contemplated according to the disclosure. The vector may comprise regulatory sequences functional in the cells desired to express the inhibitor or IL-18. Such regulatory sequences may be promoters or enhancers, for example. The regulatory sequence may then be introduced into the right locus of the genome by homologous recombination, thus operably linking the regulatory sequence with the gene, the expression of which is required to be induced or enhanced. The technology is usually referred to as "endogenous gene activation" (EGA), and it is described e.g. in WO 91/09955.

It will be understood by the person skilled in the art that it is also possible to shut down IL-18 expression using the same technique, i.e. by introducing a negative regulation element, like e.g. a silencing element, into the gene locus of IL-18, thus leading to down-regulation or prevention of IL-18 expression. The person skilled in the art will understand that such down-regulation or silencing of IL-18 expression has the same effect as the use of an IL-18 inhibitor in order to prevent and/or treat disease.

The disclosure further relates to the use of a cell that has been genetically modified to produce an inhibitor of IL-18 in the manufacture of a medicament for the treatment and/or prevention of liver injury, arthritis or inflammatory bowel disease.

The disclosure further relates to pharmaceutical compositions, particularly useful for prevention and/or treatment of inflammatory arthritis, liver injury or inflammatory bowel disease, which comprise a therapeutically effective amount of an inhibiter of IL-18 and a therapeutically effective amount of an interferon. As inhibitor of IL-18, the composition may comprise caspase-1 inhibitors, antibodies against IL-18, antibodies against any of the IL-18 receptor subunits, inhibitors of the IL-18 signalling pathway, antagonists of IL-18 which compete with IL-18 and block the IL-18 receptor, and IL-18 binding proteins, isoforms, muteins, fused proteins, functional derivatives, active fractions or circularly permutated derivatives thereof having the same activity.

IL-18BP and its isoforms, muteins, fused proteins, functional derivatives, active fractions or circularly permutated derivatives as described above are the preferred active ingredients of the pharmaceutical compositions.

The interferon comprised in the pharmaceutical composition is preferably IFN-β. In yet another preferred embodiment, the pharmaceutical composition comprises therapeutically effective amounts of an IL-18 inhibitor, optionally an interferon, and a TNF antagonist. The TNF antagonists may be antibodies neutralising TNF activity, or soluble truncated TNF receptor fragments, also called TBPI and TPBII. The pharmaceutical composition according to the disclosure may further comprise one or more COX inhibitors, preferably COX-2 inhibitors.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the disclosure can be administered to an individual in a variety of ways. The routes of administration include intradermal, transdermal (e.g. in slow release formulations), intramuscular, intraperitoneal, intravenous, subcutaneous, oral, epidural, topical, and intranasal routes. Any other therapeutically efficacious route of administration can be used, for example absorption through epithelial or endothelial tissues or by gene therapy wherein a DNA molecule encoding the active agent is administered to the patient (e.g. via a vector) which causes the active agent to be expressed and secreted in vivo. In addition, the protein(s) according to the disclosure can be administered together with other components of biologically active agents such as pharmaceutically acceptable surfactants, excipients, carriers, diluents and vehicles.

For parenteral (e.g. intravenous, subcutaneous, intramuscular) administration, the active protein(s) can be formulated as a solution, suspension, emulsion or lyophilised powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the disclosure can also be ameliorated by using conjugation procedures which increase the half-life of the molecule in the human body, for example linking the molecule to polyethylenglycol, as described in the PCT Patent Application WO 92/13095.

The therapeutically effective amounts of the active protein(s) will be a function of many variables, including the type of antagonist, the affinity of the antagonist for IL-18, any residual cytotoxic activity exhibited by the antagonists, the route of administration, the clinical condition of the patient (including the desirability of maintaining a non-toxic level of endogenous IL-18 activity

A "therapeutically effective amount" is such that when administered, the IL-18 inhibitor results in inhibition of the biological activity of IL-18. The dosage administered, as single or multiple doses, to an individual will vary depending upon a variety of factors, including IL-18 inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

According to the disclosure, the inhibitor of IL-18 is used in an amount of about 0.0001 to 10 mg/kg or about 0.01 to 5 mg/kg or body weight, or about 0.01 to 5 mg/kg of body weight or about 0.1 to 3 mg/kg of body weight or about 1 to 2 mg/kg of body weight. Further preferred amounts of the IL-18 inhibitors are amounts of about 0.1 to 1000µg/kg of body weight or about 1 to 100 µg/kg of body weight or about 10 to 50 µg/kg of body weight

The route of administration which is preferred according to the disclosure is administration by subcutaneous route. Intramuscular administration is further preferred according to the disclosure.

In further preferred embodiments, the inhibitor of IL-18 is administered daily or every other day.

The daily doses are usually given in divided doses or in sustained release form effective to obtain the desired results. Second or subsequent administrations can be performed at a dosage which is the same, less than or greater than the initial or previous dose administered to the individual. A second or subsequent administration can be administered during or prior to onset of the disease.

According to the disclosure, the IL-18 inhibitor can be administered prophylactically or therapeutically to an individual prior to, simultaneously or sequentially with other therapeutic regimens or agents (e.g. multiple drug regimens), in a therapeutically effective amount, in particular with an interferon and/or a TNF antagonist and/or a COX inhibitor. Active agents that are administered simultaneously with other therapeutic agents can be administered in the same or different compositions.

The disclosure further relates to a method for the preparation of a pharmaceutical composition comprising admixing an effective amount of an IL-18 inhibitor and/or an interferon and/or a TNF antagonist and/or a COX inhibitor with a pharmaceutically acceptable carrier.

Having now described the disclosure, it will be more readily understood by reference to the following examples that are provided by way of illustration and are not intended to be limiting of the present invention.

### EXAMPLES

### EXAMPLE 1: Production of IL-18BP-His tag

Purified recombinant human IL18BP containing a his-tag (r-hlL-18BP-His tag) was produced in CHO cells. The production of recombinant proteins in eukaryotic cells is known by the person skilled in the art. Well known methods are available for constructing appropriate vectors, carrying DNA that codes for IL-18BP and suitable for transfection of eukaryotic cells in order to produce recombinant IL-18BP. For expression in cells, the DNA coding for IL-18BP (see, e.g. (Novick et al., 1999) is cut out and inserted into expression vectors suitable for transfection of cells. Alternatively, such DNA can be prepared by PCR with suitable sense and antisense primers. The resulting cDNA constructs are then inserted into appropriately constructed eukaryotic expression vectors by techniques well known in the art (Manaitis, 1982). The recombinant protein was purified to over 95% purity and found to be biologically active in-vitro and in-vivo with a high affinity to its ligand.

### EXAMPLE 2: Protective effect of IL18BP against endotoxin-induced death in the murine model

A murine model was used to test whether IL18BP, an inhibitor of IL-18, would protect mice against a high dose of lipopolysaccharides (LPS). LPS elicits acute liver injury, followed by rapid death of the mice.

4 mg/kg of recombinant, human IL-18BP (rhIL18BPhis) containing a his-tag (resulting from recombinant production of the protein) was injected intraperitoneally (i.p). into C57BU6 mice. 1 h later, 60 mg/kg LPS were injected (lethal doses). The survival of mice was compared to a group of animals who received LPS alone (no IL18BP).

Five out of 7 mice injected with rhlL-18BP-his survived the LPS injection in contrast to the control mice, in which all animals died within 3 days.

Blood samples were taken 5 h after the LPS injection in the absence or presence of increasing doses of rhIL-18BP-his and analyzed by ELISA for circulating IFN-γ (Fig. 1). 0,4 and 4 mg/kg rhIL-18BP induced a 2 fold reduction in serum IFN-γ. This inhibition was lost at lower doses of rhIL-18BP (0,004 and 0,4 mg/kg).

### EXAMPLE 3: IL18BP has a protective effect against liver injury in a murine model of disease

A mouse model of fulminant hepatitis was used to test the effect of IL18BP. Mice develop acute liver injury when subjected to a sequential administration of Propionibacterium acnes (P. acnes) and lipopolysaccharide (LPS).

Mice were injected with increasing doses of rhIL-188P-his (4; 0,4 ; 0,04; 0 mg/kg) at various times (1 h, 20 min, simultaneously) before the injection of LPS in C57BU6 P. acnes sensitized mice. When rhIL-18BP-his was given i.p. at the same time as LPS, none of the mice survived and levels of circulating IFN-γ and TNF-α were unaffected. Surprisingly, rhIL-18BP (4 and 0.4 mg/kg) induced a 70% reduction of circulating Alanine aminotransferase (a marker of liver injury), as shown in Fig. 2.

In addition to this, the survival of mice was monitored (Fig. 3): When rhIL-18BP was given i.p. 20 minutes before LPS, the two highest doses of IL-18BP (4 and 0,4 mg/kg) delayed the death of the mice by 10 has compared to the control mice who received NaCl instead of IL-18BP.

The results of the measurement of serum IFN-γ levels are shown in Fig. 4. rhIL-18BP (4 mg/kg) inhibited 90 % of circulating IFN-γ levels and 80% of circulating Alanine aminotransferase (not shown).

When rhIL-18BP-his was given 1 h before LPS, survival curves and levels of circulating IFN-γ were similar to what was observed when rhIL-18BP-his was given 20 min before LPS, but levels of circulating Alanine aminotransferase were unaffected (not shown).

In addition to this, murine liver tissue was analyzed by hematoxilin-eosine staining, as well as by tunnel microscopy. The livers of mice, in which severe hepatitis had been induced before, showed severe necrosis as compared to normal liver tissue. In contrast to this, liver tissue of mice treated with IL-18BP showed significantly less necrotic foci than untreated mice.

### EXAMPLE 4: Anti-IL-18 antibodies protect against lethal endotoxemia

In order to evaluate, whether blockade of IL-18 with IL-18 antibodies would protect mice against lethal doses of bacterial lipopolysaccharides, C57BU6J mice were first injected with a neutralizing rabbit anti-mouse IL-18 antibody (polyclonal) or normal rabbit serum (NDS) as a control. 30 min after antibody treatment, a lethal dosis of LPS derived either from E. coli (Fig. 5 A) or S. thyphimurium (Fig. 5 B) was injected. Experiments involved 10 - 12 mice/group, and were performed twice on two different occasions.

As shown in Fig. 5 A, treatment of the mice with the anti-IL-18 antiserum prevented the mortality induced by 40 mg/kg E. coli LPS. 100 % of the mice survived after anti-IL-18 treatment vs. 10 % survival in mice treated with normal rabbit serum (p< 0,005).

Fig. 5 B shows that the antibody treated mice were also protected against S. typhimurium lethal effects (50 % vs. 0 % survival; p < 0,05).

### EXAMPLE 5: Blockade of IL-18 and TNF-α protects mice from ConA- and PEA-induced hepatotoxicity

Two experimental models of hepatotoxicity were used to evaluate the role of IL-18 and TNF-α in liver injury. Injection of Concanavalin A (Con A) and Pseudomonas aeruginosa (PEA) into mice both induce liver injury, and are models of T cell mediated hepatitis.

C57BL/6J mice were pretreated with an anti-IL-18 antiserum or a soluble TNF-α receptor, TNFsRp55. Serum Alanine aminotransferase (ALT) levels were measured as indicators of hepatic injury (Fig. 6).

As shown in Fig. 6 A, both IL-18 antiserum and soluble TNF-receptors significantly reduced ConA-induced serum ALT levels, as compared to a control injection of the vehicle alone (pyrogen free saline). A co-adminstration of soluble TNF receptor and IL-18 antiserum led to a complete inhibition of Con-A induced liver injury.

As shown in Fig. 6 B, in PEA-injected mice, neutralization of either TNF-α inhibitors or anti-IL-18 antibodies resulted in 93 % and 83 % inhibition of serum ALT levels, respectively. A combined blockade of both resulted in 99 % protection.

### EXAMPLE 6: Plasma levels of IL-18-binding protein are elevated in patients with chronic liver disease

IL-18 BP plasma levels were measured in 133 patients with chronic liver disease (CLD) of varying etiologies and 31 healthy controls by a specific ELISA, using an IL-18BP monoclonal antibody.

Plasma levels of IL-18 BP were significantly higher in CLD patients (12.91 ± 0.89 ng/ml; average ± SEM) than in healthy subjects (4.96 ± 0.43 ng/ml, p<0.001). Cirrhotic patients had signifcantly higher levels than patients with non-cirrhotic CLD (19.23 ± 1.28 ng/ml, n=67, vs. 6.49 ± 0.51 ng/ml, n=66, p<0.001). Patients with stage B of the Child-Pugh classification had higher levels of IL-18 BP than those with stage A (22.148 ± 2.44 ng/ml vs. 9.57 ± 1.25 ng/ml, p<0.001). However, there was no significant difference between Child B and C (22.48 ± 2.44 ng/ml vs. 20.62 ± 4.75 ng/ml, p=0.7). Plasma levels of IL-18 BP correlated positively with GOT, bilirubin and erythrocyte sedimentation rate. Negative correlation was found with prothrombin time.

In conclusion, the results show that IL-18 BP plasma levels are elevated in CLD and correlate with the severity of disease independent of the etiology of disease. Although an endogenous antagonist of the proinflammatory IL-18, increased levels of IL-18 BP seem not to be sufficient to counteract the overwheiming proinflammatory mediators in CLD.

### EXAMPLE 7: Inhibition of alcoholic hepatitis by IL-18BP

Four groups of rats (5 per group) are fed ethanol and a diet containing corn oil by intragastric infusion for 4 weeks. Dextrose isocalorically replaces ethanol in control rats. The rats are injected daily with mouse IL-18BP (1 mg/kg), or saline. Pathological analysis is performed on liver sections and measurements of liver enzymes in serum, TNF-α, Fas ligand and IFN-γ are taken. Necroinflammatory injury and expression of liver enzymes, TNF-α, Fas ligand, and IFN-γ are seen in the ethanol-fed rats that were injected with saline.

Rats injected with mouse IL-18BP are protected from necroinflammatory injury and the levels of liver enzymes, TNF-α, Fas ligand and IFN-γ are significantly reduced (>90%).

### EXAMPLE 8: Inhibition of Concanavalin A-induced hepatitis by IL-18BP

Balb/c mice are injected with 12 mg/kg Concanavalin A (Con A) with or without injection of murine IL-18BP (1 mg/kg), 2 h prior to Con A administration and then daily. Liver damage is evaluated by determining serum levels of liver enzymes, TNF-α, Fas ligand and IFN-γ. Hepatic histopathology is compared to mice treated with Concanavalin A only.

Pretreatment with IL-18BP significantly reduces serum levels of liver enzymes and TNF-α with no evidence of inflammation in histopathologic examination compared to control mice treated with Con A.

### REFERENCES

1. Afford, S.C., et al., Distinct patterns ofchemokine expression are associated with leukocyte recruitment in alcoholic hepatitis and alcoholic cirrhosis. J Pathol, 1998. 186(1): p. 82-9.
2. Anderson, D.M., et al., A homologue of the TNF receptor and itsligand enhance T-cell growth and dendritic-cell function. Nature, 1997. 390(6656): p. 175-179.
3. Baroni, G.S., et al., Hepatic stellate cell activation and liver fibrosis are associated with necroinflammatory injury and Th1-like response in chronic hepatitis C. Liver, 1999. 19(3): p. 212-9.
4. Bird, G.L., et al., Increased plasma tumor necrosis factor in severe alcoholic hepatitis. Ann Intern Med, 1990. 112(12): p. 917-20.
5. Bollon, D. P., et al. (1980) J. Clin. Hematol. Oncol. 10:39-48.
6. Botstein, D., et al. (1982) Miami Wint. Symp. 19:265-274.
7. Broach, J. R., in "The Molecular Biology of the Yeast Saccharomyces: Life Cycle and Inheritance", Cold Spring Harbor Laboratory, Cold Spring Harbor, NY, pp. 445-470 (1981).
8. Broach, J. R., (1982) Cell 28:203-204.
9. Bym R. A. et al., 1990, Nature (London) 344:667-670.
10. Camoglio L, te Velde AA, de Boer A, ten Kate FJ, Kopf M, van Deventer SJ. Hapten-induced colitis associated with maintained Th1 and inflammatory responses in IFN-gamma receptor-deficient mice. Eur J Immunol 2000;30:1486-95.
11. Car, B. D., V. M. Eng, B. Schnyder, L. Ozmen, S. Huang, P. Gallay, D. Heumann, M. Aguet, and B. Ryffel. 1994. Interferon gamma receptor deficient mice are resistant to endotoxic shock. J. Exp. Med. 179:1437-44 issn: 0022-1007.
12. Chater, K. F. et al., in "Sixth International Symposium on Actinomycetales Biology", Akademiai Kaido, Budapest, Hungary (1986), pp. 45-54).
13. Conti, B., J. W. Jahng, C. Tinti, J. H. Son, and T. H. Joh. 1997. Induction of interferon-gamma inducing factor in the adrenal cortex. J. Biol. Chem. 272:2035-2037.
14. Dao, T., K. Ohashi, T. Kayano, M. Kurimoto, and H. Okamura. 1996. Interferon-gamma-inducing factor, a novel cytokine, enhances Fas ligand-mediated cytotoxicity of murine T helper 1 cells. Cell-immunol. 173:230-5 issn: 0008-8749.
15. Dayer, J-M (1999). J. Cin. Inv. 104, 1337-1339.
16. Desreumaux P, Brandt E, Gambiez L, Emilie D, Geboes K, Klein O, Ectors N, Cortot A, Capron M, Colombel JF. Distinct cytokine patterns in early and chronic ileal lesions of Crohn's disease. Gastroenterology 1997;113:118-126.
17. DiDonato, J A, Hayakawa, M, Rothwarf, D M, Zandi, E and Karin, M. (1997), Nature 388, 16514-16517.
18. Elliott, M.J., Maini, R.N., Feldmann, M., Long-Fox, A., Charles, P., Bijl, H., and Woody, J.N., 1994, Lancet 344,1125-1127.
19. Engelmann, H., D. Aderka, M. Rubinstein, D. Rotman, and D. Wallach. 1989. A tumor necrosis factor-binding protein purified to homogeneity from human urine protects cells from tumor necrosis factor toxicity. J. Biol. Chem. 264:11974-11980.
20. Engelmann, H., D. Novick, and D. Wallach. 1990. Two tumor necrosis factor-binding proteins purified from human urine. Evidence for immunological cross-reactivity with cell surface tumor necrosis factor receptors. J. Biol. Chem. 265:1531-1536.
21. Fantuzzi, G., et al., IL-18 regulation of IFN-g production and cell proliferation as revealed in interleukin-1b converting enzyme-deficient mice. Blood, 1998. 91: p. 2118-2125.
22. Fiore, G., et al., Liver tissue expression of CD80 and CD95 antigens in chronic hepatitis C: relationship with biological and histological disease activities. Microbios, 1999. 97(386): p. 29-38
23. Galle, P.R., et al., Involvement of the CD95 (APO-1/Fas) receptor and ligand in liver damage. J Exp Med, 1995. 182(5): p. 1223-30.
24. Gracie, A J, Forsey, R J, Chan, W L, Gilmour, A, Leung, B P, Greer, A R, Kennedy, K, Carter, R, Wei, X-Q, Xu, D., Field, M, Foulis, A, Liew, F Y, and McInnes, I B.(1999). J. Clin. Inv. 104: 1393-1401
25. Grantham (1974), Science, 185. 862-864.
26. Grove, J., et al., Association of a tumor necrosis factor promoter polymorphism with susceptibility to alcoholic steatohepatitis [see comments]. Hepatology, 1997. 26(1): p. 143-6.
27. Gryczan, T., "The Molecular Biology of the Bacilli", Academic Press, NY (1982), pp. 307-329).
28. Gutkind, J.S., et al., A novel c-fgr exon utilized in Epstein-Barr virus-infected B lymphcytes but not in mormal monocytes. Molec. Cell. Biol., 1991. 11: p. 1500-1507.
29. Harada, K., et al., In situ nucleic acid hybridization ofcytokines in primary biliary cirrhosis: predominance of the Th1 subset. Hepatology, 1997. 25(4): p. 791-6.
30. Heremans, H., J. Van Damme, C. Dillen, R. Dijkmans, and A. Billiau. 1990. Interferon gamma, a mediator of lethal lipopolysaccharide-induced Shwartzman-like shock reactions in mice. J. Exp. Med. 171:1853-69 issn: 0022-1007.
31. Hill, D.B., et al., Increased plasma interleukin-6 concentrations in alcoholic hepatitis. J Lab Clin Med, 1992. 119(5): p. 547-52.
32. Hill, D.B., L.S. Marsano, and C.J. McClain, Increased plasma interleukin-8 concentrations in alcoholic hepatitis. Hepatology, 1993. 18: p. 576-580.
33. Hiramatsu, N., et al., Immunohistochemical detection of Fas antigen in liver tissue of patients with chronic hepatitis C. Hepatology, 1994. 19(6): p. 1354-9.
34. Huang, Y.S., et al., Serum levels of interleukin-8 in alcoholic liver disease: relationship with disease stage, biochemical parameters and survival. J Hepatol, 1996. 24(4): p. 377-84.
35. lio, S., et al., Serum levels of soluble Fas antigen in chronic hepatitis C patients. J Hepatol, 1998. 29(4): p. 517-23.
36. Izaki, K. (1978) Jpn. J. Bacteriol. 33:729-742).
37. John, J. F., et al. (1986) Rev. Infect. Dis. 8:693-704).
38. Kahiwamura, S., Okamura, H. (1998), Nippon. Rinsho. 56, pp. 1798-1806.
39. Kendall, K. J. et al. (1987) J. Bacteriol. 169:4177-4183).
40. Kim SH, Eisenstein M, Reznikov L, Fantuzzi G, Novick D, Rubinstein M, Dinarello CA. Structural requirements of six naturally occurring isoforms of the IL-18 binding protein to inhibit IL-18. Proc Natl Acad Sci U S A 2000;97:1190-1195.
41. Knight DM, Trinh H, Le J, Siegel S, Shealy D, McDonough M, Scallon B, Moore MA, Vilcek J, Daddona P, et al. Construction and initial characterization of a mouse-human chimeric anti-TNF antibody.Mol Immunol 1993 Nov 30:16 1443-53
42. Kohno, K., J. Kataoka, T. Ohtsuki, Y. Suemoto, I. Okamoto, M. Usui, M. Ikeda, and M. Kurimoto. 1997. IFN-gamma-inducing factor (IGIF) is a costimulatory factor on the activation of Th1 but not Th2 cells and exerts its effect independently of IL-12. J. Immunol. 158:1541-1550.
43. Lee, M., et al., Expression of Th1 and Th2 type cytokines responding to HBsAg and HBxAg in chronic hepatitis B patients. J Korean Med Sci, 1999. 14(2): p. 175-81.
44. Lukkari, T.A., et al., Short-term ethanol exposure increases the expression of Kupffer cell CD14 receptor and lipopolysaccharide binding protein in rat liver. Alcohol Alcohol, 1999. 34(3): p. 311-9.
45. Luo, K.X., et al., In situ investigation of Fas/FasL expression in chronic hepatitis B infection and related liver diseases. J Viral Hepat, 1997. 4(5): p. 303-7.
46. Maliszewski, C. R., T. A. Sato, T. Vanden Bos, S. Waugh, S. K. Dower, J. Slack, M. P. Beckmann, and K. H. Grabstein. 1990. Cytokine receptors and B cell functions. I. Recombinant soluble receptors specifically inhibit IL-1- and IL-4-induced B cell activities in vitro. J. Immunol. 144:3028-3033.
47. Maniatis, T., in "Cell Biology: A Comprehensive Treatise, Vol. 3: Gene Expression", Academic Press, NY, pp. 563-608 (1980).
48. Maniatis et al., Molecular Cloning: A Laboratory Manual, Cold Spring Harbor Laboratory, New York, 1982.
49. Martinez, F., et al., Ethanol and cytokine secretion. Alcohol, 1992. 9(6): p. 455-8.
50. McClain, C.J., et al., Tumor necrosis factor and alcoholic liver disease. Alcohol Clin Exp Res, 1998. 22(5 Suppl): p. 248S-252S.
51. McClain, C.J. and D.A. Cohen, Increased tumor necrosis factor production by monocytes in alcoholic hepatitis. Hepatology, 1989. 9(3): p. 349-51.
52. Micallef, M. J., T. Ohtsuki, K. Kohno, F. Tanabe, S. Ushio, M. Namba, T. Tanimoto, K. Torigoe, M. Fujii, M. Ikeda, S. Fukuda, and M. Kurimoto. 1996. Interferon-gamma-inducing factor enhances T helper 1 cytokine production by stimulated human T cells: synergism with interleukin-12 for interferon-gamma production. Eur-J-Immunol 26:1647-51 issn: 0014-2980.
53. Mizushima, S. and Nagata, S. (1990) pEF-BOS, a powerful mammalian expression vector. Nucleic Acid Res. 18:5322-5328.
54. Monteleone G, Trapasso F, Parrello T, Biancone L, Stella A, Iuliano R. Luzza F, Fusco A, Pallone F. Bioactive IL-18 expression is up-regulated in Crohn's disease. J Immunol 1999;163:143-147.
55. Muhl H, Kampfer H, Bosmann M, Frank S, Radeke H, Pfeilschifter J. Interferon-gamma mediates gene expression of IL-18 binding protein in nonleukocytic cells. Biochem Biophys Res Commun 2000;267:960-963.
56. Nakamura, K., H. Okamura, M. Wada, K. Nagata, and T. Tamura. 1989. Endotoxin-induced serum factor that stimulates gamma interferon production. Infect-Immun 57:590-5 issn: 0019-9567.
57. Nanji, A.A., et al., Activation of nuclear factor κ B and cytokine imbalance in experimental alcoholic liver disease in the rat. Hepatology, 1999. 30(4): p. 934-43.
58. Nishimura, T. and A. Ohta, A critical role for antigen-specific Th1 cells in acute liver injury in mice. J. Immunol, 1999. 162: p. 6503-6509.
59. Novick, D., B. Cohen, and M. Rubinstein. 1994. The Human Interferon alpha/beta Receptor - Characterization and Molecular Cloning. Cell 77:391-400.
60. Novick, D., B. Cohen, and M. Rubinstein. 1992. Soluble Interferon-alpha Receptor Molecules Are Present in Body Fluids. FEBS Lett 314:445-448.
61. Novick, D., H. Engelmann, D. Wallach, and M. Rubinstein. 1989. Soluble cytokine receptors are present in normal human urine. J. Exp. Med. 170:1409-1414.
62. Novick, D, Kim, S-H, Fantuzzi, G, Reznikov, L, Dinarello, C, and Rubinstein, M (1999). Immunity 10, 127-136.
63. Ohlinger, W., et al., Immunohistochemical detection of tumor necrosis factor-α, other cytokines and adhesion molecules in human livers with alcoholic hepatitis. Virchows Arch A Pathol Anat Histopathol, 1993. 423(3): p. 169-76.
64. Okamura, H., H. Tsutsui, T. Komatsu, M. Yutsudo, A. Hakura, T. Tanimoto, K. Torigoe, T. Okura, Y. Nukada, K. Hattori, K. Akita, M. Namba, F. Tanabe, K. Konishi, S. Fukuda, and M. Kurimoto. 1995. Cloning of a new cytokine that induces IFN-gamma production by T cells. Nature 378:88-91.
65. Okamoto, T., et al., Induction of Fas ligand and Fas antigen mRNA expressions in interferon-γ transgenic mouse liver. Jpn J Pharmacol, 1998. 78(2): p. 233-5.
66. Okazaki, M., et al., Hepatic Fas antigen expression before and after interferon therapy in patients with chronic hepatitis C. Dig Dis Sci, 1996. 41(12): p. 2453-8.
67. Okamoto, T., K. Yamamura, and O. Hino, The mouse interferon-γ transgene chronic hepatitis model (Review). Int J Mol Med, 1999. 3(5): p. 517-20.
68. Olee T, Hashimoto S, Quach J, Lotz M. (1999). J Immunol 162:2 1096-100
69. Pamet, P, Garka, K E, Bonnert, T P, Dower, S K, and Sims, J E. (1996), J. Biol. Chem. 271, 3967-3970.
70. Plater-Zyberk C, Bonnefoy JY. Marked amelioration of established Collagen-induced arthritis by treatment with antibodies to CD23 in vivo. Nat Med 1995;1:781-785.
71. Pizarro TT, Michie MH. Bentz M, Woraratanadharm J, Smith MF, Jr., Foley E, Moskaluk CA, Bickston SJ, Cominelli F. IL-18, a novel immunoregulatory cytokine, is up-regulated in Crohn's disease: expression and localization in intestinal mucosal cells. J Immunol 1999;162:6829-6835.
72. Reimund JM, Wittersheim C, Dumont S, Muller CD, Baumann R, Poindron P, Duclos B. Mucosal inflammatory cytokine production by intestinal biopsies in patients with ulcerative colitis and Crohn's disease. J Clin Immunol 1996;16:144-150.
73. Rothe, H., N. A. Jenkins, N. G. Copeland, and H. Kolb. 1997. Active stage of autoimmune diabetes is associated with the expression of a novelcytokine, IGIF, which is located near Idd2. J-Clin-Invest 99:469-74 issn: 0021-9738.
74. Saha N, Moldovan F, Tardif G, Pelletier JP, Cloutier JM, Martel-Pelletier J.(1999). Arthritis Rheum 42:8 1577-87.
75. Sambrook, J., E.F. Fritsch, and M. T., Molecular Cloning: A laboratory manual. 2nd ed. ed. 1989, Cold Spring Harbor, New York: Cold Spring Harbor Laboratory.
76. Simonet, W.S., et al., Osteoprotegerin: a novel secreted protein involved in the regulation of bone density. Cell, 1997. 89(2): p. 309-319.
77. Sheron, N., et al., Elevated plasma interteukin-6 and increased severity and mortality in alcoholic hepatitis. Clin Exp Immunol, 1991. 84(3): p. 449-53.
78. Sompayrac, L.H. and K.L. Danna, Efficient infection of monkey cells with DNA of simian virus 40. Proc. Nat'l. Acad. Sci. USA, 1981. 78: p. 7575-7578.
79. Sparks, C.A., et al., Assignment of the nuclear mitotic apparatus protein NuMA gene to human chromosome 11q13. Genomics, 1993. 17: p. 222-224.
80. Su, G.L., et al., CD14 and lipopolysaccharide binding protein expression in a rat model of alcoholic liver disease. Am J Pathol, 1998. 152(3): p. 841-9.
81. Taieb, J., et al., Raised plasma soluble Fas and Fas-ligand in alcoholic liver disease [letter]. Lancet, 1998. 351(9120): p. 1930-1.
82. Triantaphyllopoulos, K A, Williams, R, Tailor, H, and Chernakovsky, Y (1999). Arthritis and Rheumatism 42, 90-99.
83. Tsutsui, H., K. Nakanishi, K. Matsui, K. Higashino, H. Okamura, Y. Miyazawa, and K. Kaneda. 1996. IFN-gamma-inducing factor up-regulates Fas ligand-mediated cytotoxic activity of murine natural killer cell clones. J. Immunol. 157:3967-73 issn: 0022-1767.
84. Tsuij, H., Mukaida, N., Harada A., Kaneko, S., Matsushita, E., Nakanuma, Y., Tsutsui, H., Okamura, H., Nakanishi, K., Tagawa,m Y, Iwakura, Y., Kobayashi, K., and Matsuschima, K.(1999), J. Immunol. 162, pp. 1049-1055.
85. Tucci, A., James, H., Chicheportiche, R., Bonnefoy, J.Y., Dayer, J.M., and Zubler, R.H., 1992, J. Immunol. 148, 2778-2784.
86. Ushio, S., M. Namba, T. Okura, K. Hattori, Y. Nukada, K. Akita, F. Tanabe, K. Konishi, M. Micallef, M. Fujii, K. Torigoe, T. Tanimoto, S. Fukuda, M. Ikeda, H. Okamura, and M. Kurimoto. 1996. Cloning of the cDNA for human IFN-gamma-inducing factor, expression in Escherichia coli, and studies on the biologic activities of the protein. J. Immunol. 156:4274-4279.34. Okayama, H. and Berg, P. (1983) A cDNA cloning vector that permits expression of cDNA inserts in mammalian cells. Mol. Cell. Biol. 3:280-289.
87. Williams RO, Mason LJ, Feldmann M, Maini RN. Synergy between anti CD4 and anti-tumor necrosis factor in the amelioration of established collagen-induced arthritis. Proc Natl Acad Sci U S A 1994 Mar 29 91:7 2762-6.
88. Yasuda, H., et al., Identity of osteoclastogenesis inhibitory factor (OCIF) and osteoprotegerin (OPG): a mechanism by which OPGIOCIF inhibits osteoclastogenesis in vitro. Endocrinology, 1998. 139: p. 1329-1337.
89. Yoshimoto T, Takeda, K, Tanaka, T, Ohkusu, K. Kashiwamura, S, Okamura, H, Akira, S and Nakanishi, K (1998). J. Immunol. 161, 3400-3407.

## Claims

1. Use of an IL-18 inhibitor selected from the group of an IL-18 antibody and an IL-18 binding protein, or an isoform, a mutein, fused protein, functional derivative, active fraction or circularly permutated derivative thereof having essentially the same activity as an IL-18 binding protein, for the manufacture of a medicament for treatment and/or prevention of chronic liver injury, selected from the group consisting of alcoholic hepatitis.

2. The use according to claim 1, wherein the IL-18 antibody is a humanised IL-18 antibody.

3. The use according to claim 1, wherein the IL-18 antibody is a human IL-18 antibody.

4. The use according to claim 1, wherein the IL-18 binding protein is PEGylated.

5. The use according to claim 1, wherein the inhibitor of IL-18 is a fused protein comprising all or part of an IL-18 binding protein fused to all or part of an immunoglobulin, and wherein the fused protein binds to IL-18.

6. The use according to claim 5, wherein the fused protein comprises all or part of the constant region of an immunoglobulin.

7. The use according to claim 6, wherein the immunoglobulin is of the IgG1 or IgG2 isotype.

8. The use according to any of the preceding claims, wherein the medicament further comprises an interferon.

9. The use according to claim 8, wherein the interferon is interferon-β.

10. The use according to claim 8 or 9, wherein the inhibitor of IL-18 is used simultaneously, sequentially, or separately with the interferon.

11. The use according to any of the preceding claims, wherein the medicament further comprises a Tumor Necrosis Factor (TNF) antagonist.

12. The use according to claim 11, wherein the TNF antagonist is a soluble TNF-receptor I (TBPI) and/or a soluble TNF-receptor II (TBPII).

13. The use according to claim 11 or 12, wherein the inhibitor of IL-18 and/or the interferon is used simultaneously, sequentially, or separately with the TNF antagonist.

14. The use according to any of the preceding claims, wherein the medicament further comprises a COX-inhibitor.

15. The use according to claim 14, wherein the COX-inhibitor is a COX-2 inhibitor.

16. The use according to any of the preceding claims, wherein the inhibitor of IL-18 is used in an amount of about 0.0001 to 10 mg/kg of body weight, or about 0.01 to 5 mg/kg of body weight or about 0.1 to 3 mg/kg of body weight or about 1 to 2 mg/kg of body weight.

17. The use according to any of the preceding claims, wherein the inhibitor of IL-18 is used in an amount of about 0.1 to 1000 µg/kg of body weight or 1 to 100 µg/kg of body weight or about 10 to 50 µg/kg of body weight.

18. The use according to any of the preceding claims, wherein the IL-18 inhibitor is administered subcutaneously.

19. The use according to any of the preceding claims, wherein the IL-18 inhibitor is administered intramuscularly.

20. The use according to any of the preceding claims, wherein the IL-18 inhibitor is administered daily.

21. The use according to any of the preceding claims, wherein the IL-18 inhibitor is administered every other day.

22. Use of an expression vector comprising the coding sequence of IL-18 binding protein for the manufacture of a medicament for the treatment and/or prevention of chronic liver injury selected from the group consisting of alcoholic hepatitis.

23. Use according to claim 22 for gene therapy.

24. Use of a cell that has been genetically modified to produce an inhibitor of IL-18 for the manufacture of a medicament for the treatment and/or prevention of chronic liver injury selected from the group consisting of alcoholic hepatitis.

## Patentansprüche

1. Verwendung eines IL-18 Inhibitors ausgewählt aus der Gruppe bestehend aus einem IL-18 Antikörper und einem IL-18 Bindungsprotein, oder einer Isoform, einem Mutein, einem Fusionsprotein, einem funktionellen Derivat, einer aktiven Fraktion oder einem zirkulär permutierten Derivat davon mit im wesentlichen der gleichen Aktivität wie ein IL-18 Bindungsprotein, zur Herstellung eines Medikaments zur Behandlung und/oder Vorbeugung von chronischer Lebererkrankung ausgewählt aus der Gruppe bestehend aus Alkohol-Hepatitis.

2. Verwendung nach Anspruch 1, wobei der IL-18 Antikörper ein humanisierter IL-18 Antikörper ist.

3. Verwendung nach Anspruch 1, wobei der IL-18 Antikörper ein humaner IL-18 Antikörper ist.

4. Verwendung nach Anspruch 1, wobei das IL-18 Bindungsprotein PEGyliert ist.

5. Verwendung nach Anspruch 1, wobei der Inhibitor von IL-18 ein Fusionsprotein ist, welches das ganze IL-18 Bindungsprotein oder einen Teil davon, fusioniert an ein ganzes Immunglobulin oder einen Teil davon, umfasst, und wobei das Fusionsprotein an IL-18 bindet.

6. Verwendung nach Anspruch 5, wobei das Fusionsprotein das Ganze oder einen Teil der konstanten Region eines Immunglobulins umfasst.

7. Verwendung nach Anspruch 6, wobei das Immunglobulin vom IgG1 oder IgG2 isotyp ist.

8. Verwendung nach einem der vorstehenden Ansprüche, wobei das Medikament weiterhin ein Interferon umfasst.

9. Verwendung nach Anspruch 8, wobei das interferon Interferon-β ist.

10. Verwendung nach Anspruch 8 oder 9, wobei der Inhibitor von IL-18 und das interferon gleichzeitig, hintereinander oder getrennt verwendet werden.

11. Verwendung nach einem der vorstehenden Ansprüche, wobei das Medikament weiterhin einen Tumomekrosefaktor (TNF)-Antagonist umfasst.

12. Verwendung nach Anspruch 11, wobei der TNF-Antagonist ein löslicher TNF-Rezeptor I (TBPI) und/oder ein löslicher TNF-Rezeptor II (TBPII) ist.

13. Verwendung nach Anspruch 11 oder 12, wobei der Inhibitor von IL-18 und/oder das Interferon gleichzeitig, hintereinander oder getrennt von dem TNF-Antagnoist verwendet werden.

14. Verwendung nach einem der vorstehenden Ansprüche, wobei das Medikament weiterhin einen COX-Inhibitor umfasst.

15. Verwendung nach Anspruch 14, wobei der COX-Inhibitor ein COX-2-Inhibitor ist.

16. Verwendung nach einem der vorstehenden Ansprüche, wobei der Inhibitor von IL-18 in einer Menge von etwa 0,0001 bis 10 mg/kg Körpergewicht, oder etwa 0,01 bis 5 mg/kg Körpergewicht, oder etwa 0,1 bis 3 mg/kg Körpergewicht, oder etwa 1 bis 2 mg/kg Körpergewicht verwendet wird.

17. Verwendung nach einem der vorstehenden Ansprüche, wobei der Inhibitor von IL-18 in einer Menge von etwa 0,1 bis 1000 µg/kg Körpergewicht, oder 1 bis 100 µg/kg Körpergewicht, oder etwa 10 bis 50 µg/kg Körpergewicht verwendet wird.

18. Verwendung nach einem der vorstehenden Ansprüche, wobei der IL-18 Inhibitor subkutan verabreicht wird.

19. Verwendung nach einem der vorstehenden Ansprüche, wobei der IL-18 Inhibitor intramuskulär verabreicht wird.

20. Verwendung nach einem der vorstehenden Ansprüche, wobei der IL-18 Inhibitor täglich verabreicht wird.

21. Verwendung nach einem der vorstehenden Ansprüche, wobei der IL-18 Inhibitor jeden zweiten Tag verabreicht wird.

22. Verwendung eines Expressiorisvektors, umfassend die kodierende Sequenz des IL-18 Bindungsproteins zur Herstellung eines Medikaments zur Behandlung und/oder Vorbeugung einer chronischen Lebererkrankung ausgewählt aus der Gruppe bestehend aus Alkohol-Hepatitis.

23. Verwendung nach Anspruch 22 zur Gentherapie.

24. Verwendung einer Zelle, die gentechnisch modifiziert wurde, um einen Inhibitor des IL-18 zu produzieren, zur Herstellung eines Medikaments zur Behandlung und/oder Vorbeugung einer chronischen Lebererkrankung ausgewählt aus der Gruppe bestehend aus Alkohol-Hepatitis.

## Revendications

1. Utilisation d'un inhibiteur de l'interleukine IL-18 choisi dans l'ensemble formé par un anticorps anti-IL-18 et une protéine se liant à l'IL-18, ou une isoforme, une mutéine, une protéine de fusion, un dérivé fonctionnel, une fraction active ou un dérivé par permutation circulaire d'une telle protéine, doté(e) d'une activité qui est pratiquement la même que celle d'une protéine se liant à l'IL-18, en vue de la fabrication d'un médicament conçu pour le traitement et/ou la prévention d'une lésion chronique du foie choisie dans l'ensemble formé par l'hépatite alcoolique.

2. Utilisation conforme à la revendication 1, dans laquelle l'anticorps anti-IL-18 est un anticorps anti-IL-18 humanisé.

3. Utilisation conforme à la revendication 1, dans laquelle l'anticorps anti-IL-18 est un anticorps anti-IL-18 humain.

4. Utilisation conforme à la revendication 1, dans laquelle la protéine se liant à l'IL-18 est pegylée.

5. Utilisation conforme à la revendication 1, dans laquelle l'inhibiteur de l'IL-18 est une protéine de fusion, comprenant tout ou partie d'une protéine se liant à l'IL-18, fusionnée avec tout ou partie d'une immunoglobuline, laquelle protéine de fusion se lie à l'IL-18.

6. Utilisation conforme à la revendication 5, dans laquelle la protéine de fusion comprend tout ou partie d'une région constante d'une immunoglobuline.

7. Utilisation conforme à la revendication 6, dans laquelle l'immunoglobuline appartient à l'isotype IgG1 ou IgG2.

8. Utilisation conforme à l'une des revendications précédentes, le médicament devant en outre comprendre un interféron.

9. Utilisation conforme à la revendication 8, pour laquelle l'interféron est de l`interféron bêta.

10. Utilisation conforme à la revendication 8 ou 9, l'inhibiteur de l'IL-18 devant être utilisé simultanément ou séquentiellement avec l'interféron ou séparément d'avec celui-ci:

11. Utilisation conforme à l'une des revendications précédentes, le médicament devant comprendre en outre un antagoniste du facteur TNF (facteur de nécrose de tumeurs).

12. Utilisation conforme à la revendication 11, pour laquelle l'antagoniste du facteur TNF est un récepteur soluble de type I du TNF (récepteur TBP-I) et/ou un récepteur soluble de type II du TNF (récepteur TBP-II).

13. Utilisation conforme à la revendication 11 ou 12, l'inhibiteur de l'IL-18 et/ou l'interféron devant être utilisé(s) simultanément ou séquentiellement avec l'antagoniste du facteur TNF ou séparément d'avec celui-ci.

14. Utilisation conforme à l'une des revendications précédentes, le médicament devant comprendre en outre un inhibiteur de COX (cyclo-oxygénase).

15. Utilisation conforme à la revendication 11, pour laquelle l'inhibiteur de COX est un inhibiteur de COX-2.

16. Utilisation conforme à l'une des revendications précédentes, pour laquelle on utilise l'inhibiteur de l'IL-18 en une quantité d'environ 0,0001 à 10 mg par kg de poids corporel, ou d'environ 0,01 à 5 mg par kg de poids corporel, ou d'environ 0,1 à 3 mg par kg de poids corporel, ou d'environ 1 à 2 mg par kg de poids corporel.

17. Utilisation conforme à l'une des revendications précédentes, pour laquelle on utilise l'inhibiteur de l'IL-18 en une quantité d'environ 0,1 à 1000 µg par kg de poids corporel, ou d'environ 1 à 100 µg par kg de poids corporel, ou d'environ 10 à 50 µg par kg de poids corporel.

18. Utilisation conforme à l'une des revendications précédentes, l'inhibiteur de l'IL-18 devant être administré par voie sous-cutanée.

19. Utilisation conforme à l'une des revendications précédentes, l'inhibiteur de l'IL-18 devant être administré par voie intramusculaire.

20. Utilisation conforme à l'une des revendications précédentes, l'inhibiteur de l'IL-18 devant être administré chaque jour.

21. Utilisation conforme à l'une des revendications précédentes, l'inhibiteur de l'IL-18 devant être administré tous les deux jours.

22. Utilisation d'un vecteur d'expression comprenant une séquence codant une protéine se liant à l'IL-18, en vue de la fabrication d'un médicament conçu pour le traitement et/ou la prévention d'une lésion chronique du foie choisie dans l'ensemble formé par l'hépatite alcoolique.

23. Utilisation conforme à la revendication 22, pour une thérapie génique.

24. Utilisation d'une cellule qui a été génétiquement modifiée pour produire un inhibiteur de l'IL-18, en vue de la fabrication d'un médicament conçu pour le traitement et/ou la prévention d'une lésion chronique du foie choisie dans l'ensemble formé par l'hépatite alcoolique.
